# EUROPEAN PATENT APPLICATION

(11) **EP 2 332 648 A1**
(43) Date of publication of application: **15.06.2011**
(21) Application number: 09806723.4
(22) Date of filing: 11.08.2009
(51) Int. Cl.: B01J 31/02, C07C 27/00, C07C 29/00, C07C 31/04, C07C 51/00, C07C 53/02, C07D 487/22, C12P 7/04, C07B 61/00

(54) **CATALYST FOR DEGRADING LIGNIN, CATALYST FOR DEGRADING AROMATIC HYDROCARBON, AND PORPHYRIN**

(30) Priority: 11.08.2008 JP 2008238637; 24.09.2008 JP 2008272496; 14.10.2008 JP 2008288228; 24.04.2009 WO PCT/JP2009/058209
(71) Applicant: Fukutome, Hirofumi, Fukuoka 815-0083 (JP)
(72) Inventor: ISHIBASHI Toru, Nagasaki-shi Nagasaki 852-8155 (JP)
(74) Representative: AMMANN PATENTANWÄLTE AG BERN
(86) International application number: PCT/JP2009/064201
(87) International publication number: WO 2010/018832

(57) **Abstract**

Herein provided are a lignin-decomposition catalyst and an aromatic hydrocarbon-decomposition catalyst, which contain a porphyrin. Alcohols and organic acids can be isolated or separated from lignin by adding a solution containing an alkaline compound to lignin, optionally acting a lignin-decomposition catalyst on the resulting lignin-alkaline compound-containing solution and optionally irradiating, with light rays, the solution; or by optionally acting a lignin- decomposition catalyst on lignin and then irradiating it with light rays. The decomposition products generated during the isolation of the foregoing alcohols and organic acids are recovered and hydrogen ions are released from lignin. Herein provided also include a porphyrin having such a catalytic function that it can convert lignin into alcohols and organic acids; a porphyrin having such a catalytic function that it can decompose a compound containing an aromatic hydrocarbon in which an oxygen is linked to a carbon atom constituting the benzene ring thereof; and a porphyrin obtained by cultivating *Escherichia coli* which cannot express the gene: ypjD (b2611) due to the variation thereof.

## Description

### [Technical Field]

The present invention relates to a catalyst for the decomposition of lignin, a method for the preparation of alcohols and organic acids, a method for the preparation of lignin decomposition products, a catalyst for the decomposition of an aromatic hydrocarbon, a method for the release of hydrogen ions, and a porphyrin and more particularly to a lignin decomposition catalyst comprising a porphyrin, a method for preparing alcohols such as methanol and organic acids starting from lignin, a method for preparing lignin decomposition products, an aromatic hydrocarbon decomposition catalyst comprising a porphyrin, a method for making hydrogen ions release from lignin using a lignin decomposition catalyst comprising a porphyrin, and a porphyrin.

### [Prior Art]

The global warming has increasingly been progressed since the beginning of the 21^{st} century and the reduction of emitted carbon dioxide becomes a key to controlling the industrial world and, in its turn, the economy of the world. In as much as using fossil fuels enclosed in the underground and/or the sea floor or bed as an energy source, it would be difficult not only to reduce the amount of carbon dioxide in the atmosphere, but also to control any increase in the amount thereof. Under such circumstances, attracted special interest recently are alcohols such as bio-ethanol prepared from plants and they are full of promising as a further energy source. However, in this case, most of the conventional techniques for preparing such bio-ethanol make use of sugar or sugars as starting materials and accordingly, a problem arises such that the resource conflict occurs between the mankind's foods and the energy. Recently, there have been developed, at long last, advanced techniques for preparing alcohols while making use of carbon sources such as celluloses, which never cause confliction with the sources of the foods for the humankind.

The woods and the grass or weeds, which cannot be used as foodstuffs for the humankind, in principal, consist of cellulose and lignin. If using, as carbon sources, cellulose materials originated from woods which are almost considered as waste materials and unsuitable for use as construction materials, such as scrap wood and chip-like materials as well as grass or weeds, the amount of the carbon dioxide discharged into the environmental atmosphere is inhibited and thus this may considerably make a contribution to the industrial world and the economic world.

Contrary to the foregoing progress in the usage of cellulose, the effective use of lignin, which has been considered as an abundant carbon resource like the cellulose, has still been limited only to considerably narrow fields. As an example of such effective use of lignin, which has already been put into practical use, the lignin is simply combusted as a heat source and it has been used as an antiseptic or as a structure-reinforcing material by incorporating it into concrete.

Moreover, along with the advance of the scientific technology and the industries, the industrial waste regarded as compounds, which cannot originally be present in a high concentration in the natural world, would be accumulated therein and the accumulation thereof may become a serious negative property for the humankind. Most of these compounds are ones containing aromatic hydrocarbons in which an oxygen atom is linked to a carbon atom constituting the benzene ring thereof. Examples thereof include dioxins. The problem of generating dioxins can be solved to a considerable degree by a treatment of dioxins at a high temperature in the combustion step of garbage as a source of the dioxins, but a large quantity of harmful compounds may have already been diffused into the environmental atmosphere. For this reason, if these harmful compounds can be decomposed into harmless ones with the use of a harmful compound decomposition catalyst which is, by nature, present in the natural world, while making use of the solar rays infinitely poured on the surface of the earth, this would permit the purification or cleansing of the earthly environment and contribute to the maintenance of the health of the mankind. However, there has not yet been proposed any such a useful decomposition catalyst.

In addition, the fuel battery in which an electromotive force is generated through the movement of hydrogen ions has become of maj or interest recently, as an energy source. When using hydrogen gas as a hydrogen ion source, however, most of the hydrogen gas as a source of hydrogen ions has presently been produced from the fossil fuel. Moreover, hydrogen gas can likewise be produced through the electrolysis of water, but an electric power should be supplied even in this case.

Furthermore, in the case of a solar battery in which an electric power is generated through the use of the solar rays, a semiconductor device should be produced and enormous amounts of natural resources and a vast cost are required for the production of such a semiconductor device if the recent demands for the energy should be supplied by the use of such solar batteries. Even in the case of dye-sensitized type solar batteries, titanium oxide of a nano size should be used and a synthetic dyestuff is considerably expensive, which can provide an electromotive force to a certain extent.

There has conventionally been known a method for decomposing a lignin-containing substance which comprises the step of bringing the lignin-containing substance into close contact with functional water to thus decompose the substance (see, for instance, Patent Document 1 specified below). This conventional technique discloses that the functional water includes sodium hydroxide, but there is not any distinct or specific disclosure about the decomposition products and the technique does not disclose that alcohols can be obtained through the decomposition. In addition, it has also been known that porphyrins containing a plurality of Cl and F atoms are active in a reaction such as the oxidation of lignin and the conversion of an alkane into an alcohol (see, for instance, Patent Document 2 specified below). However, this conventional technique does not include any disclosure concerning the use of any alkaline compound and any photocatalyst.

### [Prior Art Documents]

[Patent Document 1] Japanese Un-Examined Patent Publication 2000-144592 (claims, the passage included in the section [0030]);
[Patent Document 2] TOKUHYO Hei 2-503086 (the passage appearing in the upper left column on page 4).

### [Summary of the Invention]

### [Problems That the Invention is to Solve]

It is an object of the present invention to provide a lignin decomposition catalyst comprising a porphyrin, which has never been used in the conventional techniques; a method for preparing alcohols such as methanol and organic acids, which makes use of lignin as a starting material; a method for preparing lignin decomposition products; a catalyst for decomposing an aromatic hydrocarbon, which comprises a porphyrin; a method for releasing hydrogen ions using lignin as a starting material; and a porphyrin.

### [Means for the Solution of the Problems]

The catalyst for the decomposition of lignin according to the present invention is characterized in that it comprises a porphyrin which can show its catalytic function through the irradiation thereof with light rays.

The lignin decomposition catalyst according to the present invention is characterized in that it comprises a porphyrin which can show its catalytic function in an alkaline solution.

The lignin decomposition catalyst according to the present invention is characterized in that it comprises a porphyrin which can show its catalytic function in an alkaline solution, while irradiating the same with light rays.

The lignin decomposition catalyst according to the present invention is characterized in that the porphyrin is a tetrapyrrole compound carrying a methyl group and an ethyl ester group or an acetate group (propionic acid group) on the porphyrin ring.

The foregoing catalyst for the decomposition of lignin is characterized in that the porphyrin is a tetrapyrrole compound carrying four methyl groups and four ethyl ester groups or acetate groups (propionic acid groups) on the porphyrin ring.

The foregoing catalyst for the decomposition of lignin is characterized in that the porphyrin is one having, in the molecule, a carboxyl group.

The foregoing catalyst for the decomposition of lignin is characterized in that the porphyrin is one having, in the molecule, two, four or eight carboxyl groups, in total.

The foregoing catalyst for the decomposition of lignin is characterized in that the porphyrin is at least one member selected from the group consisting of uroporphyrin, protoporphyrin and coproporphyrin.

The foregoing catalyst for the decomposition of lignin is characterized in that the porphyrin is a tetrapyrrole compound having a porphyrin ring structure, which is obtained by cultivating *Escherichia coli* in a culture medium and then isolating the same from the culture medium.

The foregoing catalyst for the decomposition of lignin is characterized in that the catalyst comprises a culture medium containing a tetrapyrrole compound having a porphyrin ring structure, which is obtained by cultivating *Escherichia coli* in a culture medium.

The foregoing catalyst for the decomposition of lignin is characterized in that the *Escherichia coli* is one which cannot express the gene ypjD (b2611) due to the variation or mutation thereof.

The foregoing catalyst for the decomposition of lignin is characterized in that the *Escherichia coli* is an insertion variant in which the transposon for the gene ypjD (b2611) is inserted.

The method for the preparation of alcohols and organic acids according to the present invention is characterized in that it comprises the steps of adding an alkaline compound-containing solution to lignin and then separating or isolating alcohols and organic acids from the resulting solution containing the lignin/alkaline compound.

The foregoing method for the preparation of alcohols and organic acids is characterized in that the solution containing lignin and the alkaline compound is irradiated with light rays (preferably irradiated with, for instance, UV light rays or solar rays) and then the alcohols and organic acids are separated or isolated from the solution containing lignin and the alkaline compound.

The foregoing method for the preparation of alcohols and organic acids is characterized in that the alcohols and organic acids are separated or isolated from the solution containing lignin and the alkaline compound, after further acting the aforementioned catalyst for the decomposition of lignin on the solution containing lignin and the alkaline compound.

The foregoing method for the preparation of alcohols and organic acids is characterized in that the alcohols and organic acids are separated or isolated from the solution containing lignin and the alkaline compound, after acting the aforementioned catalyst for the decomposition of lignin on the solution containing lignin and the alkaline compound and then further irradiating the same with light rays (preferably irradiating it with, for instance, UV light rays or solar rays).

The method for the preparation of alcohols and organic acids according to the present invention is characterized in that the aforementioned catalyst for the decomposition of lignin is acted on lignin and then the alcohols and organic acids released from the lignin are separated and isolated.

The method for the preparation of alcohols and organic acids according to the present invention is characterized in that lignin is irradiated with light rays (preferably irradiated with, for instance, UV light rays or solar rays) and then the alcohols and organic acids released from the lignin are separated and isolated.

The foregoing method for the preparation of alcohols and organic acids is characterized in that the alcohols and organic acids released from lignin are separated or isolated, after acting the aforementioned catalyst for the decomposition of lignin on the lignin and then further irradiating the same with light rays (preferably irradiating it with, for instance, UV light rays or solar rays).

The foregoing method for the preparation of alcohols and organic acids is characterized in that the alkaline compound is at least one member selected from the group consisting of KOH and NaOH.

The foregoing method for the preparation of alcohols and organic acids is characterized in that the alcohol is methanol and the organic acids are formic acid, acetic acid, malic acid, succinic acid and pyruvic acid.

The foregoing method for the preparation of alcohols and organic acids is characterized in that the separation of the alcohols is carried out by the distillation.

The method for the preparation of lignin decomposition products according to the present invention is characterized in that it comprises the step of recovering the lignin decomposition products (carbon-containing compounds of low molecular weight) generated during the separation of the alcohols and organic acids according to the aforementioned method for the preparation of alcohols and organic acids.

The catalyst for the decomposition of an aromatic hydrocarbon in which an oxygen atom is linked to a carbon atom constituting a benzene ring according to the present invention is characterized in that the catalyst comprises a porphyrin.

The foregoing catalyst for the decomposition of an aromatic hydrocarbon is characterized in that the porphyrin is a tetrapyrrole compound having a porphyrin ring structure, which is obtained by cultivating *Escherichia coli* in a culture medium and then isolating the compound from the medium.

The foregoing catalyst for the decomposition of an aromatic hydrocarbon is characterized in that the catalyst comprises a culture medium containing a tetrapyrrole compound having a porphyrin ring structure, which is obtained by cultivating *Escherichia coli* in a culture medium.

The foregoing catalyst for the decomposition of an aromatic hydrocarbon is characterized in that the *Escherichia coli* is one which cannot express the gene ypjD (b2611) due to the variation thereof.

The foregoing catalyst for the decomposition of an aromatic hydrocarbon is characterized in that the *Escherichia coli* is an insertion variant in which the transposon for the gene ypjD (b2611) is inserted.

The foregoing catalyst for the decomposition of an aromatic hydrocarbon is characterized in that the porphyrin is a tetrapyrrole compound carrying a methyl group and an ethyl ester group or an acetate group (propionic acid group) on the porphyrin ring.

The foregoing catalyst for the decomposition of an aromatic hydrocarbon is characterized in that the porphyrin is a tetrapyrrole compound carrying four methyl groups and four ethyl ester groups or acetate groups (propionic acid groups) on the porphyrin ring.

The foregoing catalyst for the decomposition of an aromatic hydrocarbon is characterized in that the porphyrin is at least one member selected from the group consisting of uroporphyrin, protoporphyrin, coproporphyrin and ethioporphyrin.

The foregoing catalyst for the decomposition of an aromatic hydrocarbon is characterized in that the porphyrin is one having, in the molecule, a carboxyl group.

The foregoing catalyst for the decomposition of an aromatic hydrocarbon is characterized in that the porphyrin is one carrying, in the molecule, two, four or eight carboxyl group in total.

The foregoing catalyst for the decomposition of an aromatic hydrocarbon is characterized in that the aromatic hydrocarbons are dioxins.

The method for making hydrogen ions release according to the present invention is characterized in that the method comprises the steps of acting the foregoing lignin decomposition catalyst on lignin or a solution containing lignin and an alkaline compound, irradiating the solution with light rays (preferably irradiating with, for instance, UV light rays or solar light rays) and then separating or isolating hydrogen ions released from the reaction system.

The porphyrin according to the present invention is characterized in that it has a catalytic function of converting lignin into alcohols and organic acids.

The porphyrin according to the present invention is characterized in that it has a catalytic function of decomposing a compound containing an aromatic hydrocarbon in which an oxygen atom is linked to a carbon atom constituting the benzene ring thereof.

The porphyrin according to the present invention is characterized in that it is obtained by cultivating the *Escherichia coli* which cannot express the gene ypjD (b2611) due to the variation thereof.

The catalyst for the decomposition of lignin according to the present invention is characterized in that it comprises the porphyrin obtained by cultivating the *Escherichia coli* which cannot express the gene ypjD (b2611) due to the variation thereof.

### [Effects of the Invention]

According to the present invention, porphyrin such as a tetrapyrrole compound carrying a porphyrin structure, which is biologically prepared using *Escherichia coli,* and a synthetic porphyrin can accomplish the effect as an effective catalyst for the decomposition of lignin and as an effective catalyst for the decomposition of an aromatic hydrocarbon.

According to the present invention, the alcohols and organic acids can be prepared and the low-molecular-weight decomposition products can likewise be prepared and further hydrogen ions can be separated or isolated from lignin, by acting, on lignin, an alkaline compound, the irradiation with light rays and/or the foregoing lignin decomposition catalyst, which may be used alone or in any combination.

### [Brief Description of the Drawings]

[Figure 1] Fig. 1 shows the structural formula of the product produced in Preparation Example 1.
[Figure 2] Fig. 2 shows the spectral diagram as the results of the spectroscopic analysis (absorbance) observed for the product produced in Preparation Example 1.
[Figure 3] Fig. 3 shows the ¹H NMR spectra observed for Sample 1 prepared in Preparation Example 1.
[Figure 4] Fig. 4 shows the ¹³C NMR spectra observed for Sample 1 prepared in Preparation Example 1.
[Figure 5] Fig. 5 shows the HSQC spectra observed for Sample 1 prepared in Preparation Example 1.
[Figure 6] Fig. 6 shows the COSY spectra observed for Sample 1 prepared in Preparation Example 1.
[Figure 7] Fig. 7 shows the HMBC spectra observed for Sample 1 prepared in Preparation Example 1.
[Figure 8] Fig. 8 shows the NOESY spectra observed for Sample 1 prepared in Preparation Example 1.
[Figure 9] Fig. 9 shows the NOESY spectra observed for Sample 1 prepared in Preparation Example 1.
[Figure 10] Fig. 10 shows the ¹H NMR spectra observed for Sample 2 prepared in Preparation Example 1.
[Figure 11] Fig. 11 shows the ¹³C NMR spectra observed for Sample 2 prepared in Preparation Example 1.
[Figure 12] Fig. 12 shows an enlarged NOESY spectra observed for Sample 2 prepared in Preparation Example 1.
[Figure 13] Fig. 13 shows the spectra illustrating the relative abundance observed for Sample 2 prepared in Preparation Example 1.
[Figure 14] Fig. 14 shows the results of the analysis carried out according to the ESI-MS.
[Figure 15] Fig. 15 shows the results of the analysis of the product prepared in Preparation Example 1, which is carried out according to the pyrolysis GC-MS.

### [Mode for Carrying Out the Invention]

The mode of carrying out the present invention will now be described below in detail.

According to an embodiment of the lignin decomposition catalyst and the catalyst for the decomposition of an aromatic hydrocarbon in which an oxygen atom is linked to a carbon atom constituting the benzene ring thereof, according to the present invention, these catalysts each comprise a porphyrin which can show its catalytic function through the irradiation with light rays and/or in an alkaline aqueous solution. The porphyrin is preferably a tetrapyrrole compound, which has a porphyrin ring structure and is prepared by cultivating *Escherichia coli* in a culture medium and then isolating the porphyrin secreted in the medium, or a synthetic porphyrin. For instance, these catalysts may be a culture medium containing a tetrapyrrole compound having a porphyrin ring structure and obtained by the cultivation of *Escherichia coli* in a culture medium or porphyrin per se recovered from cultivated cells or the extract from proliferated and cultivated cells.

The foregoing *Escherichia coli* is preferably one whose gene expression has been changed such as one which cannot express the gene ypjD (b2611) due to the variation or mutation thereof, or an insertion variant strain or a variant thereof, wherein the transposon for the gene ypjD (b2611) is inserted into the gene of the bacteria. The foregoing porphyrin is more preferably a tetrapyrrole compound carrying a methyl group (for instance, four methyl groups) and an ethyl ester group or an acetate group (propionic acid group) (for instance, four ethyl ester groups or acetate groups) on the porphyrin ring.

The *Escherichia coli*-derived lignin decomposition and aromatic hydrocarbon decomposition catalysts according to the present invention can be prepared according to, for instance, the method detailed below:

The culture medium used for the production of the foregoing catalyst is not restricted to any particular one inasmuch as it can cultivate *Escherichia coli. Escherichia coli* can be cultivated in an oligotrophic or eutrophic culture medium, and then isolating and recovering the intended tetrapyrrole compound from the culture medium to thus give the tetrapyrrole compound having a porphyrin ring structure, which can constitute the lignin decomposition catalyst and the catalyst for the decomposition of an aromatic hydrocarbon. Thus, the tetrapyrrole compound can be prepared by making *Escherichia coli* produce the same during the process for the cultivation and proliferation of the bacterial cells and subsequently recovering the tetrapyrrole compound secreted in the culture medium. To prevent any influence of components such as natural substances present in the culture medium, which may be an obstacle to the isolation of the tetrapyrrole compound, it is preferred to use an oligotrophic culture medium, but the present invention is not restricted to the use thereof. Preferably used as such oligotrophic culture mediums include, for instance, culture mediums each containing glucose or lactose, but the present invention is not restricted to the use thereof.

The *Escherichia coli* used in the production of the lignin decomposition catalyst and the aromatic hydrocarbon decomposition catalyst is preferably one which cannot express the gene ypjD (b2611) due to the variation thereof. Specific examples thereof include *Escherichia coli* cells derived from K12 strains and BL21 strains. For instance, preferably used herein are *Escherichia coli* cells derived from K12 strains, which cannot express the gene ypjD (b2611) due to the variation thereof. Examples of *Escherichia coli* strains which cannot express the gene ypjD (b2611) through the mutation include *Escherichia coli* strains wherein the transposon for the gene ypjD (b2611) is inserted into the gene of the strains. Such mutant strains are in such a condition that the function thereof for the expression of the gene ypjD (b2611) is partially or completely deleted. In this respect, the K12 strains are available from, for instance, National Bio-Resources, while the B21 strains are available from, for instance, TAKARA BIO. Moreover, the variant strains wherein the transposon for the gene ypjD (b2611) is inserted into the gene thereof include, for instance, JD23504 strains available from National Bio-Resources.

In this embodiment, *Escherichia coli* strains are first cultivated in an oligotrophic culture medium. In this respect, it is preferred that the *Escherichia coli* strains are pre-cultured in an appropriate culture medium other than the oligotrophic culture medium such as LB culture medium and that the pre-cultivated strains (or pre-cultivated products) are subsequently inoculated into an oligotrophic culture medium to thus carry out principal cultivation. In addition, it may also be possible to use a eutrophic culture medium and synthesized cultivation liquid in addition to the oligotrophic culture medium. For instance, it may be a synthesized cultivation liquid, in the form of an aqueous solution, prepared by adding, for instance, KH₂PO₄, K₂HPO₄, (NH₄)₂SO₄, citric acid dihydrate, glucose and MgSO₄ to deionized water. In any case, the culture medium to be used is not restricted to any specific one insofar as it can be used for growing or proliferating *Escherichia coli* strains.

The conditions for growing *Escherichia coli* are not limited to any particular ones and the strains thereof can be cultivated under those currently used for the proliferation thereof. The same would be true for the following case, i.e., when *Escherichia coli* strains are first pre-cultured and then the principal cultivation thereof is carried out while replacing the culture medium with another one. For instance, the bacterial cells are pre-cultured in LB culture medium, at a temperature ranging from 15 to 40°C for 6 to 24 hours and then the resulting cell suspension is subjected to the principal cultivation in an oligotrophic culture medium at a temperature ranging from 20 to 40°C for 12 to 96 hours. Thus, the bacterial cells proliferate or grow in the culture medium and as a result, a culture (cultivated product) can be obtained, which has a color tone peculiar to the intended tetrapyrrole compound.

Then, the intended tetrapyrrole compound can be isolated from the foregoing cultivated product according to the method detailed below.

More specifically, the cultured product is centrifuged to give a supernatant followed by the filtration thereof, and then the tetrapyrrole compound is isolated from the filtrate through adsorption using, for instance, a column packed with an ion-exchange resin or a reversed phase column. For instance, the culture medium obtained after the cultivation of the bacterial cells is centrifuged to thus make the bacterial cells sediment or precipitate to thus give a supernatant containing culture (cultured product). Then the supernatant is filtered through a filter having a predetermined pore size (for instance, 0.22µm) and then the resulting filtrate is loaded on the foregoing column packed with an ion-exchange resin to thus adsorb the intended products on the resin. Thereafter, the cultured product is eluted from the ion-exchange resin using, for instance, 20% acetonitrile-0.1% trifluoroacetic acid solution and then the resulting eluate is lyophilized. In this respect, it is also possible to use an eluting solution prepared by adding an aqueous solution of an acid or an alkali to an organic solvent in the foregoing elution step. According to this embodiment, one or at least two kinds of tetrapyrrole compounds are obtained and several milligrams to several ten-milligrams of tetrapyrrole compounds can be obtained from, for instance, 500mL of the cell suspension.

The product isolated according to the foregoing procedures can be analyzed by, for instance, the NMR (Nuclear Magnetic Resonance) spectroscopic measurement to thus confirm whether or not the tetrapyrrole compounds are present in the product. In addition, when analyzing the product according to the spectrophotometric measurement, it would be found that the compounds are ones having an absorption peak within a wavelength region peculiar to the dyestuffs. In most of cases, those compounds are dyestuff compounds each showing a bilateral peak similar to those observed for chlorophyll, heme or phthalocyanine. Such dyestuff compounds are useful as photocatalysts or electron-transfer materials whose electrons are excited through the irradiation thereof with light rays. Moreover, they are involved in the reduction-oxidation (redox) reaction in an aqueous solution or through the cell membrane and accordingly, it would be believed that they can likewise function in a battery.

As has been discussed above in detail, tetrapyrrole compounds, for instance, porphyrins such as porphine and porphyrins can be produced using *Escherichia coli* and therefore, this method never requires the use of production devices and catalysts selected depending on the kinds of the intended compounds unlike the production thereof according to the chemical synthesis method, the former method does not require the use of any solvent and accordingly, it is only slightly apprehended that the method adversely affects the surrounding environment. In addition, it is not needed to add any precursor for the tetrapyrrole compound such as 5-aminolevulinic acid to the culture medium when cultivating *Escherichia coli* (see Japanese Un-Examined Patent Publication Hei 5-244937), and further it is sufficient to recover the tetrapyrrole compounds secreted in the culture medium and it is not necessary to collect the same from the bacterial cell (see Japanese Un-Examined Patent Publication Hei 5-91866). In other words, the method used in the present invention does not require the use of any particular compound and device for the cultivation of *Escherichia coli* and the recovery of the resulting tetrapyrrole compounds and accordingly, the method permits the easy production of the tetrapyrrole compounds. The tetrapyrrole compounds thus prepared can be used in a variety of industrial fields such as the medical care, food and electronics.

In the foregoing, the tetrapyrrole compounds are isolated from the culture medium or cultured products to thus give the lignin decomposition catalyst and the aromatic hydrocarbon decomposition catalyst. More specifically, it is preferred to use, as such compounds, those recovered from the cultured product, but the cultured product and the bacterial cells obtained through the cultivation contain tetrapyrrole compounds and therefore, the cultured product per se or the bacterial cells per se can likewise be used as the lignin decomposition catalyst and the aromatic hydrocarbon decomposition catalyst.

As the lignin decomposition catalyst and the aromatic hydrocarbon decomposition catalyst usable in the present invention, there can be listed as a synthetic porphyrin, for instance, at least one member selected from the group consisting of protoporphyrin, uroporphyrin, coproporphyrin and ethioporphyrin, in addition to the foregoing ones, provided that the ethioporphyrin among others never decomposes lignin. In the following Examples, there were used, as such porphyrins, Protoporphyrin IX (available from ALDRICH Company) carrying, in the molecule, two carboxyl groups; Uroporphyrin I (available from SIGMA Company) carrying 8 carboxyl groups in the molecule; Coproporphyrin I (available from ALDRICH Company) carrying 4 carboxyl groups in the molecule; and Ethioporphyrin (available from ALDRICH Company) free of any carboxyl group in the molecule. This porphyrin is also free of any coordinated transition metal at the center of the porphyrin ring, like the foregoing porphyrins obtained through the culture or proliferation of *Escherichia coli.*

Then the embodiments of the method for preparing alcohols and organic acids according to the present invention will be described below in detail. According to the present invention, alcohols and organic acids can be prepared by acting, on lignin, an alkaline compound, the irradiation with light rays and /or the foregoing lignin decomposition catalyst, wherein these means for decomposing lignin can be used alone or in any combination.

More specifically, alcohols such as methanol can be produced by, for instance, the following methods: (1) A method comprising the steps of adding, to lignin, an aqueous solution containing at least one alkaline compound selected from the group consisting of, for instance, KOH and NaOH, and then isolating alcohols formed and released into the lignin-alkaline compound-containing solution through, for instance, distillation; (2) a method comprising the steps of adding, to lignin, the foregoing aqueous solution containing an alkaline compound, irradiating the lignin-alkaline compound-containing solution with ultraviolet rays (examples of such ultraviolet rays are those emitted from a UV ray-emitting lamp such as ENF Type one available from SPECTRONICS Company (such as 260c/j and 280c/j) or UVL-56Hand Held available from UVH Company) and light rays having a wide wavelength range such as solar rays for a predetermined time period and efficiently isolating alcohols from the lignin-alkaline compound-containing solution, which had been irradiated with light rays, through, for instance, distillation; (3) a method comprising the steps of adding, to lignin, the foregoing aqueous solution containing an alkaline compound, acting the foregoing lignin decomposition catalyst on the lignin-alkaline compound-containing solution at a predetermined temperature for a predetermined time period, and then efficiently isolating alcohols from the lignin-alkaline compound-containing solution through, for instance, distillation; (4) a method comprising the steps of adding, to lignin, the foregoing aqueous solution containing an alkaline compound, acting the foregoing lignin decomposition catalyst on the lignin-alkaline compound-containing solution at a predetermined temperature for a predetermined time period, irradiating the lignin-alkaline compound-containing solution with UV light rays or light rays having a wide wavelength range such as solar rays for a predetermined time period and efficiently isolating alcohols from the lignin-alkaline compound-containing solution, which had been irradiated with light rays, through, for instance, distillation; (5) a method comprising the steps of acting the foregoing lignin decomposition catalyst on lignin at a predetermined temperature for a predetermined time period and then efficiently isolating alcohols from the resulting solution obtained after the catalytic reaction through, for instance, distillation; (6) a method comprising the steps of irradiating lignin-containing solution with UV light rays or light rays having a wide wavelength range such as solar rays for a predetermined time period and then efficiently isolating alcohols from the lignin-containing solution, which had been irradiated with light rays, through, for instance, distillation; or (7) a method comprising the steps of acting the foregoing lignin decomposition catalyst on lignin at a predetermined temperature for a predetermined time period, irradiating the lignin-containing solution with UV light rays or light rays having a wide wavelength range such as solar rays for a predetermined time period and then efficiently isolating alcohols from the lignin-containing solution, which had been irradiated with light rays, through, for instance, distillation. When carrying out the foregoing step of irradiating lignin with light rays, the step is preferably carried out in an atmosphere, in which the reaction solution containing lignin can come in close contact with, for instance, the air, oxygen gas or a gas containing oxygen and/or nitrogen, so that the porphyrin efficiently shows its catalytic function. For instance, when the lignin concentration and that of porphyrin are set at levels of 2.5 mg/mL and 50µg/mL (weight ratio: 50/1) respectively, it is possible to use 0.2 to 0.5 mL of oxygen gas per 1 mg of lignin.

Moreover, organic acids such as formic acid, acetic acid, malic acid, succinic acid and pyruvic acid can be separated or isolated from lignin according to the same methods described above in connection with the isolation of the alcohols.

As the lignin used in the present invention is not restricted to any specific one and usable herein include, for instance, products having high purity and free of any impurities such as a reducing sugar and cellulose (such as a product available from SIGMA Company under the Catalogue No. 471003 having a molecular weight of 60,000; a product available from ALDRICH Company under the Catalogue No. 471046 having a molecular weight of 12,000); products having a slightly low purity containing reducing sugar (such as a product available from SIGMA Company under the Catalogue No. 471038 having a molecular weight of 52,000) and products insoluble in water (such as a product available from ALDRICH Company under the Catalogue No. 370967). The present invention permits the separation or isolation of alcohols such as methanol and organic acids such as formic acid, acetic acid, malic acid, succinic acid and pyruvic acid, in amounts almost identical to one another, from all the foregoing lignin. More specifically, the present invention would permit the production of alcohols and organic acids while using lignin as a starting material, irrespective of the presence of impurities, the difference in the average molecular weight of the lignin used and the difference in the solubility thereof in water.

The solution of an alkaline compound used in the present invention is not restricted to any particular one and preferably used herein are, for instance, solutions of KOH and/or NaOH each having a concentration ranging from about 0.0025M to about 0.05M. However, the concentration of the solution of an alkaline compound is not restricted to the level falling within the range specified above, although there is observed some difference in the separation efficiency between alcohols and organic acid.

According to an embodiment of the catalyst for decomposing an aromatic hydrocarbon in which an oxygen atom is linked to a carbon atom as a member of the benzene ring, the catalyst has the same composition as that of the lignin decomposition catalyst which comprises the foregoing porphyrin and therefore, the detailed description of the former will herein be omitted.

The aromatic hydrocarbons, with which the foregoing catalyst interact, include, for instance, dioxins and compounds analogous to dioxins. Examples of such dioxins include poly(chloro-dibenzo-p-dioxin) (PCDD), poly(chloro-dibenzo-furan) (PCDF), while examples of dioxin-analogous compounds are coplanar poly(biphenyl chlorides) (dioxin-like PCBs). The aromatic hydrocarbon decomposition catalyst according to the present invention would permit the decomposition or conversion of these dioxins into harmless products.

Furthermore, according to another embodiment of the present invention, there is provided a method for releasing hydrogen ions from lignin. According to this method, hydrogen ions can be isolated from lignin by the addition of the lignin decomposition catalyst which comprises the porphyrin consisting of the foregoing pyrrole compound derived from *Escherichia coli* or a synthetic porphyrin to a lignin-alkaline compound-containing aqueous solution and this in turn permits the photolytic decomposition of lignin.

According to a still another embodiment of the present invention, low-molecular-weight carbon atom-containing compounds can be recovered, which are lignin-decompositionproducts formed in the step of isolating the alcohols and organic acids according to the foregoing method for the production of such alcohols and organic acids.

The present invention is not restricted to the embodiments described above and includes various variations thereof without departing from the gist of the present invention. The present invention will hereunder be described in more detail with reference to the following Preparation Examples and working Examples.

### (Preparation Example 1)

As has been discussed above, alcohols and organic acids can be prepared by adding, to lignin, a porphyrin (a pyrrole compound) as a lignin decomposition catalyst and further decomposition products can be obtained by the photolytic decomposition of lignin and hydrogen ions released from lignin can likewise be isolated. Moreover, tetrapyrrole compounds each having a porphyrin structure and synthetic porphyrins are effective as catalysts for decomposing an aromatic hydrocarbon such as dioxin. In this case, the porphyrin usable herein may be, for instance, those biologically prepared using *Escherichia coli.* In this Preparation Example, the preparation of a pyrrole compound will be described, which makes use of *Escherichia coli*.

The cells of an insertion variant in which the transposon for the gene ypjD (b2611) originated from *Escherichia coli* had been inserted (JD23504 available from National Bio-Resources) were cultivated in 2 mL of LB culture medium (bacto-tryptone: 1%; bacto-yeast extract: 0.5%; NaCl: 0.5%) at 37°C for 12 hours. There was added 1 mL of the resulting cell suspension to 500 mL of an aqueous solution prepared by adding 9 g of KH₂PO₄, 21 g of K₂HPO₄, 2 g of (NH₄)₂SO₄, 1 g of citric acid dihydrate, 3.6 g of glucose and 200 mg of MgSO₄ to 1 L of deionized water and the cells were subjected to principal cultivation at 37°C for 24 hours.

The cultured liquid thus obtained was found to be colorless at the beginning of the principal cultivation, but the color thereof turned pink color after the elapse of 24 hours. This cultured liquid was treated using a centrifuge to thus precipitate the cells present therein and the resulting supernatant was filtered through a filter having a pore size of 0.22µm. Then the resulting filtrate was passed through a column packed with an anion-exchange resin, subsequently the culture adsorbed on the resin was eluted from the same using a 20% acetonitrile-0.1%trifluoroacetic acid solution as an eluent and then the eluate was lyophilized. A product tinged with pink was thus recovered.

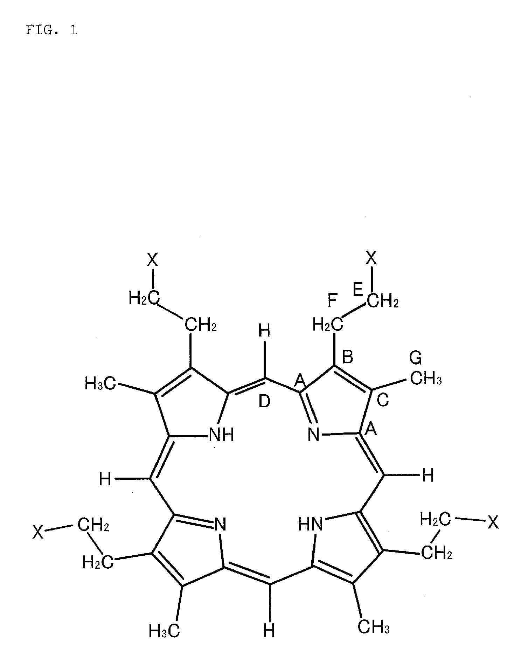

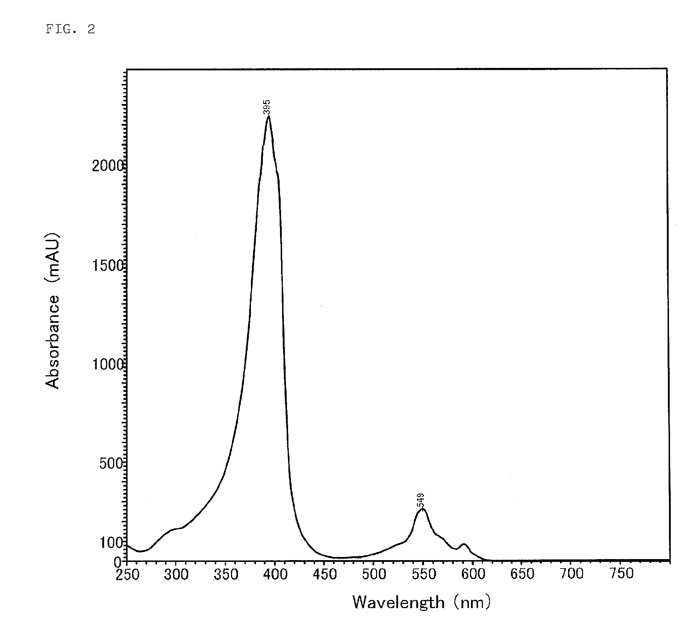

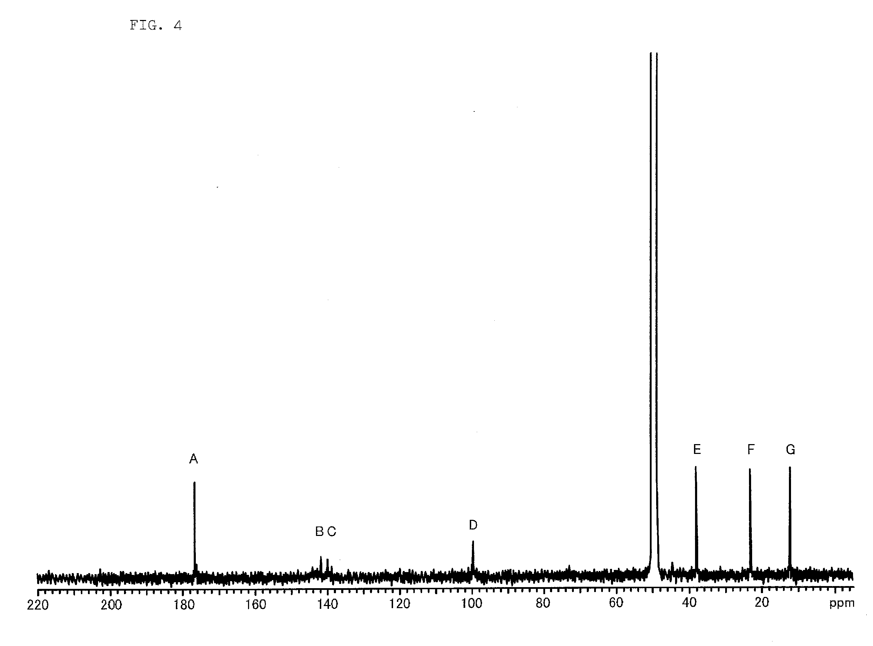

This product was subjected to a variety of instrumental analyses, such as those detailed below, and it was confirmed that the product was a tetrapyrrole compound having a structure as shown in Fig. 1. The symbols A to G appearing in Fig. 1 correspond to the marks specifying the corresponding ¹³C NMR spectral peaks as shown in Fig. 4, these in the following figures are shown in the same way also. Moreover, the product was analyzed according to the NMR spectrometry and it was confirmed that a tetrapyrrole compound was certainly present in the product. Furthermore, the presence of potassium (K) was detected by the ICP (Inductively Coupled Plasma) mass spectrometric analysis and it was thus concluded that the compound was recovered as one ionically coupled with K or in the form of a complex with the same. In addition, the product was further subjected to the spectrophotometric analysis and as a result, it was confirmed that there was observed a bilateral peak including the Soret band peculiar to the porphyrin, as shown in Fig. 2. In Fig. 2, there are observed two peaks at wavelengths of 395 nm and 549 nm, respectively. This clearly indicates that the product is an organic dyestuff carrying free electrons capable of undergoing migration.

The tetrapyrrole compound prepared above was dissolved in different solvents (Sample 1 and Sample 2), the resulting samples were subjected to the two-dimensional NMR spectrometry (COSY, NOESY, HSQC, HMBC), followed by the analysis of the resulting spectra and the structural analysis of the compound.

Regarding the sample 1, it was dissolved in CD₃OD and then the resulting solution was subjected to the NMR spectroscopic measurement under the following conditions:
- Apparatus Used: INOVA500 Model (available from Variant Company);
- Resonance Frequency: 499.8 MHz (¹H);
- Standard: 3.31 ppm (CD₂HOD, ¹H NMR)
   49.421 ppm (CD₃OD, ¹³C NMR);

- Integration Number: ¹H NMR (16 times); ¹³C NMR 53428 times); COSY (16 times); NOESY (8 times); HSQC (32 times); HMBC (128 times);
- Other Condition: The mixing time of NOESY was set at a level of 400 msec.

In addition, as to the sample 2, it was dissolved in a 90:10:0.1 mixed CD₃CN/D₂O/CD₃COOD solvent and then the resulting solution was subjected to the NMR spectroscopic measurement under the following conditions:
- Apparatus Used: INOVA600 Model (available from Variant Company);
- Resonance Frequency: 599.8 MHz (¹H);

- Standard: 1.92 ppm (CD₂HCN, ¹H NMR)
   1.28 ppm (CD₃CN, ¹³C NMR);
- Integration Number: ¹H NMR (64 times); ¹³C NMR 50000 times); COSY (16 times); NOESY (16 times); HSQC (32 times); HMBC (128 times);
- Other Condition: The mixing time of NOESY was set at a level of 400 msec.

### Structural Analysis of Sample 1:

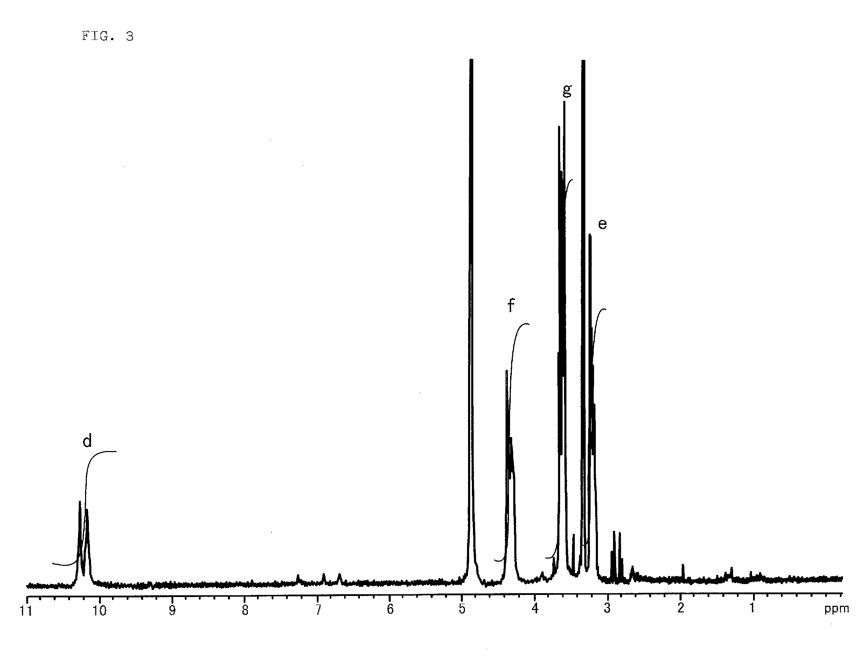

The results of the ¹H NMR spectrometric analysis are plotted on Fig. 3 (solvent: CD₃OD). As a result, there were observed signals at about 10.0 to 10.5 ppm (d), around 4.3 ppm (f), around 3.6 ppm (g), and around 3.2 ppm (e), which were assumed to be ascribed to the intended component. The signals' intensity ratio was found to be about 1:2:3:2. The signals d to g are identical to those appearing on the other figures.

The results of the ¹³C NMR spectrometric analysis are plotted on Fig. 4. It was thus assumed that the compound was an aromatic one because of the presence of signals B and C.

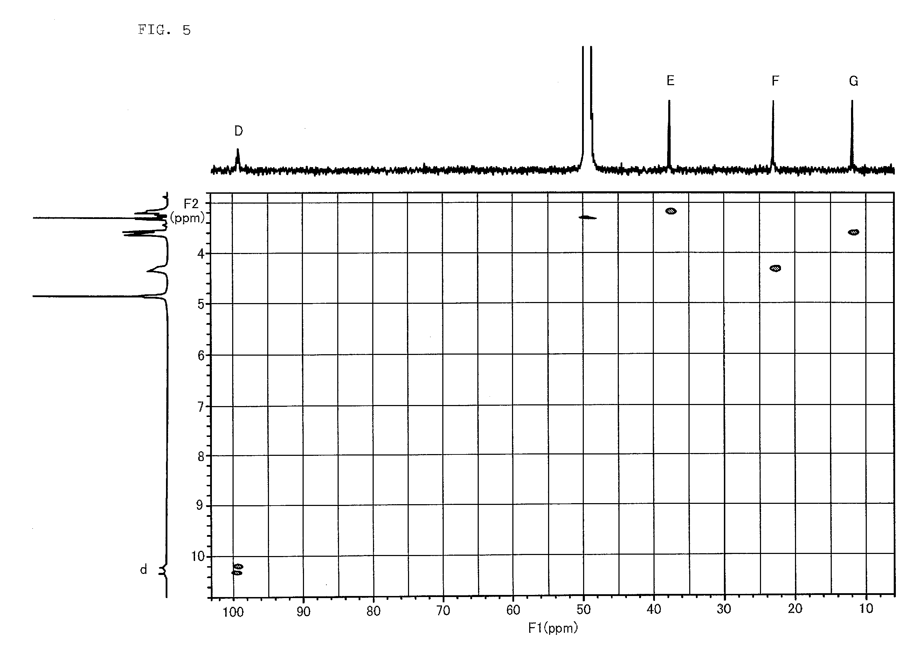

In this connection, the d signal observed in the ¹H NMR spectrometric measurement is a characteristic signal which is not currently observed in the light of the magnitude of the chemical shift and it was assumed to be ascribed to the porphyrin skeleton, as a candidate, while taking into consideration the fact that the sample compound was an aromatic one. As will be described below, when analyzing the results (Figs. 5 to 9) obtained by the two-dimensional NMR spectrometry while regarding the compound as a porphyrin, the results can be analyzed without any contradiction. Moreover, a compound having a structure similar to that estimated and depicted on Fig. 1 is reported in J. Org. Chem., 1999, Vol. 164, No. 21, pp. 7973-7982 and the magnitude of the chemical shift detected by the ¹H NMR spectrometric measurement is well consistent with that disclosed in the article. Accordingly, it can reasonably be concluded that the compound has a porphyrin structure.

The analysis of the two-dimensional NMR spectrometric measurement will be described below in detail.
The results of the HSQC spectrometric analysis are shown in Fig. 5. The HSQC spectrometry is an analytical technique for the detection of J¹_{CH}. The results thus obtained are also depicted on Fig. 5. Regarding the proton signals, capital letters are ascribed to carbon atoms directly bonded.

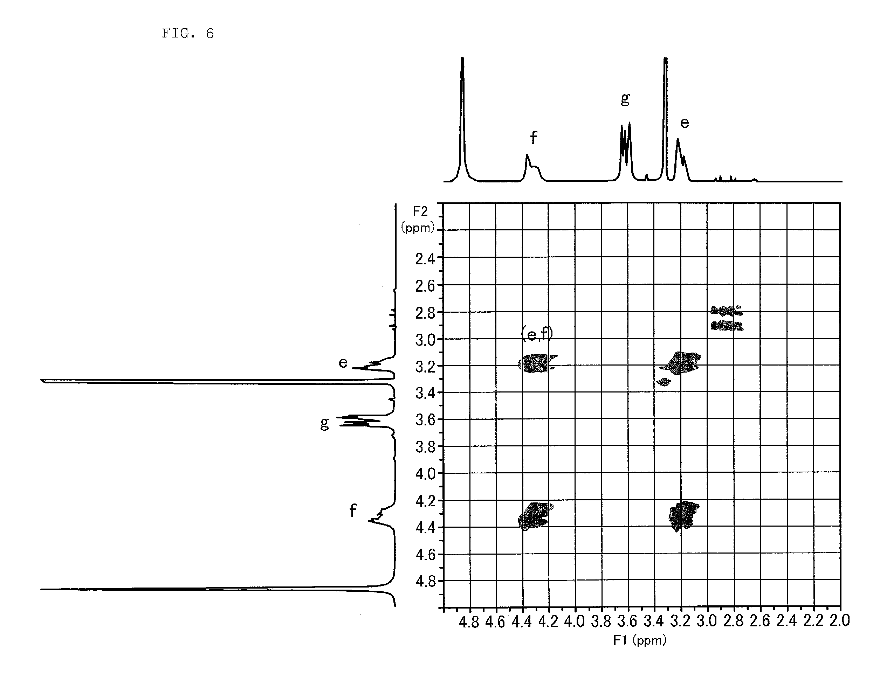

The results of the COSY spectrometric analysis are shown in Fig. 6. The COSY spectrometry is an analytical technique for the detection of the spin coupling between ¹H-¹H. As a result of the analysis, it was found that f and e caused a spin coupling and it was believed that this could be ascribed to -CH₂-CH₂-X at a high probability in consideration of the signal intensity ratio and the magnitudes of chemical shift for F and E. In this connection, X represents an unidentified component whose structure has not yet been elucidated.

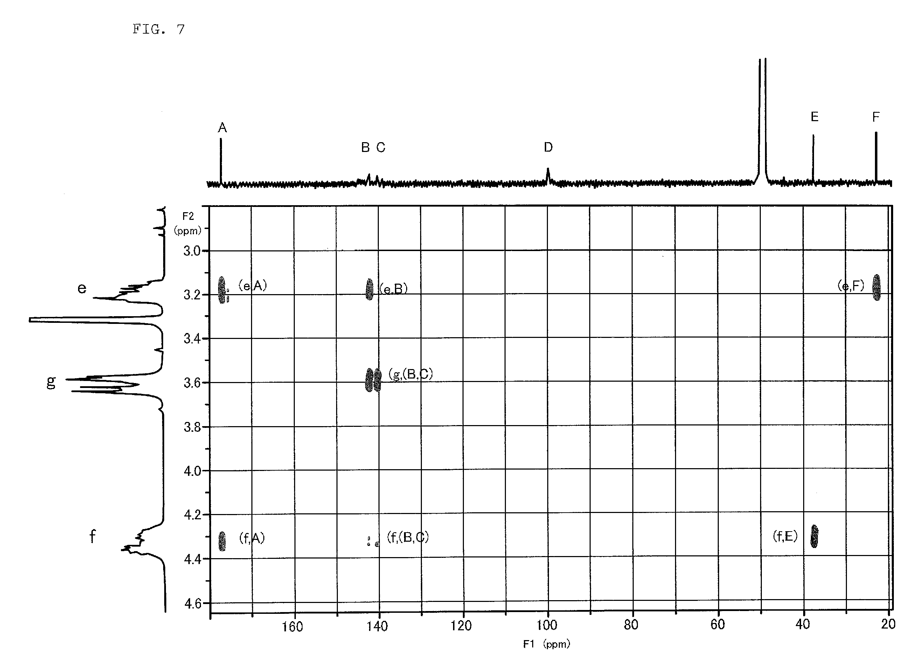

The results of the HMBC spectrometric analysis are shown in Fig. 7. The HMBC spectrometry is an analytical technique for the detection of Jⁿ_{CH} (n= about 2 to 4), and this technique provides the heterogeneous nuclear remote coupling correlated spectra. The analysis of the spectrogram clearly indicates the presence of such correlations as (e, A), (e, B), (e, F), (g, B), (g, C), (f, A), (f, B), (f, C), (f, E). These correlations are never contradictory to the estimated structure as shown in Fig. 1.

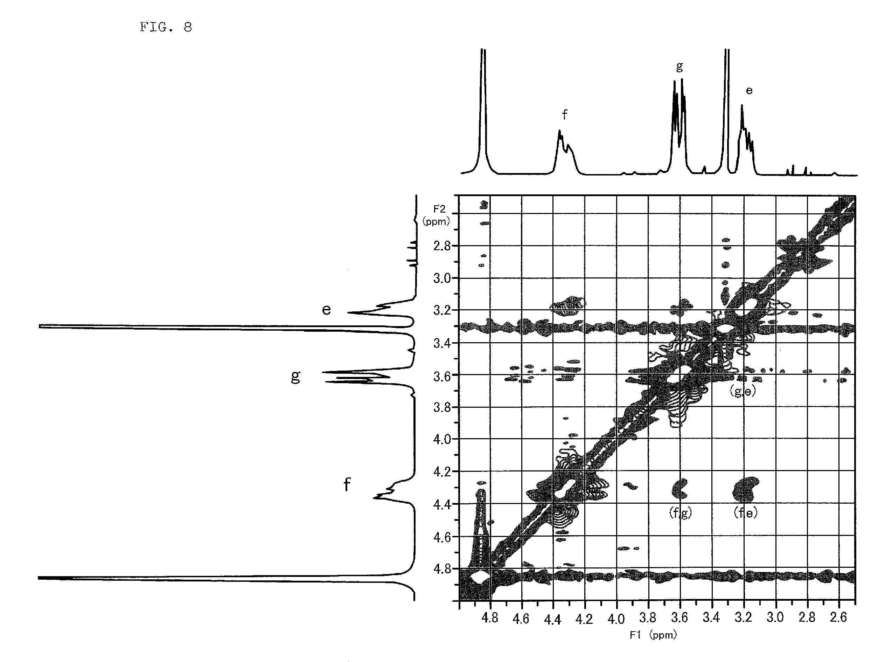

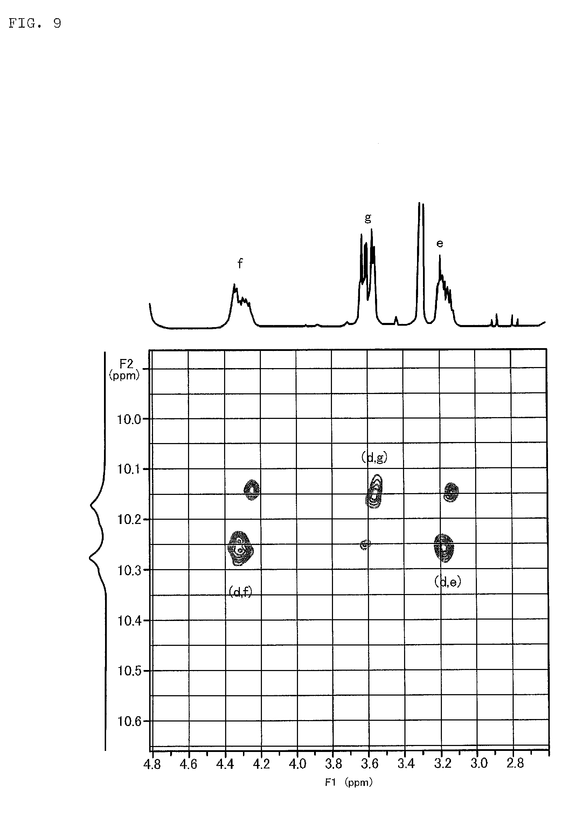

The results of the NOESY spectrometric analysis are shown in Figs. 8 and 9. The NOESY spectrometry is an analytical technique for the detection of the presence of magnetization-exchange and accordingly, this would be able to provide the information concerning the distance between nuclear spins on the basis of the magnetization shift due to the cross relaxation. The analysis of the spectrogram clearly indicates the presence of such NOE correlations as (g, e), (f, g), (f, e), (d, f), (d, e), (d, g) correlations. These NOE correlations clearly supported the reliability of the estimated structure as shown in Fig. 1.

### Structural Analysis of Sample 2:

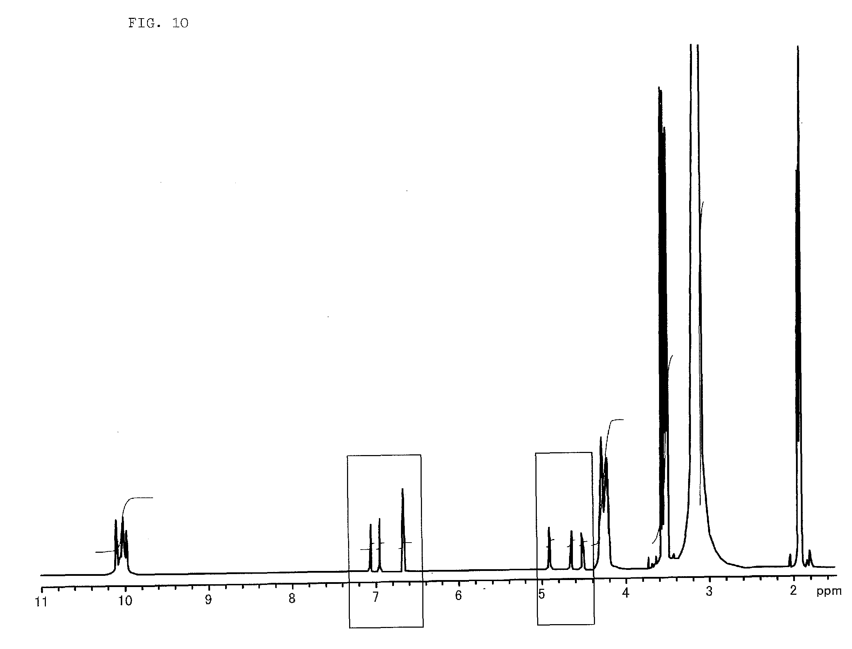

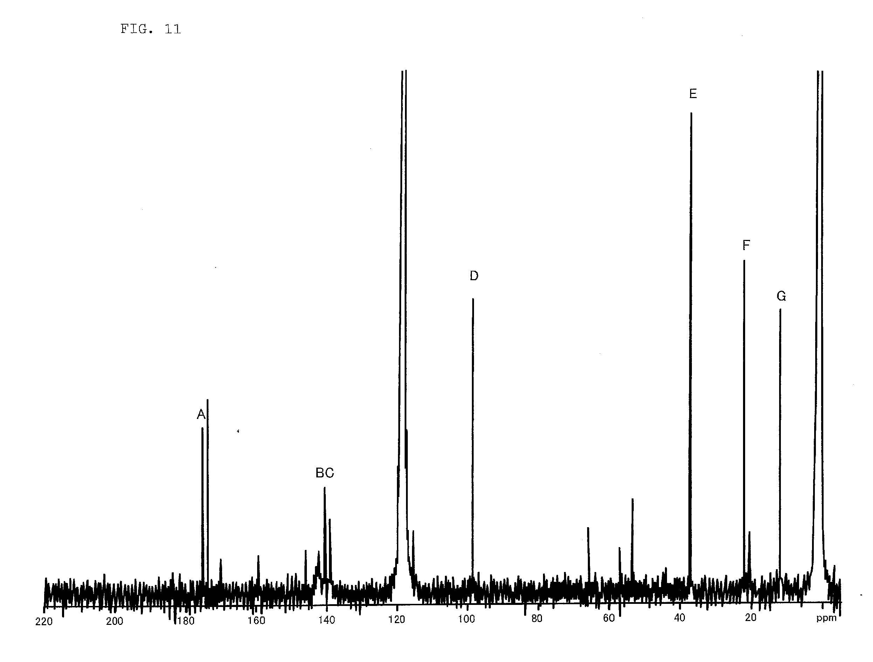

The results of the ¹H NMR spectrometric analysis are plotted on Fig. 10 and the results of the ¹³C NMR spectrometric analysis are plotted on Fig. 11 (solvent: CD₃CN/D₂O/CD₃COOD = 90:10:0.1). In Fig. 10, the signals enclosed in two squares are ascribed to impurities. As a result of the comparison with the spectra observed for the foregoing sample 1, it was assumed that the structures of the principal components were identical to one another. This was also supported by the analytical results of respective COSY, NOESY, HSQC and HMBC spectrograms.

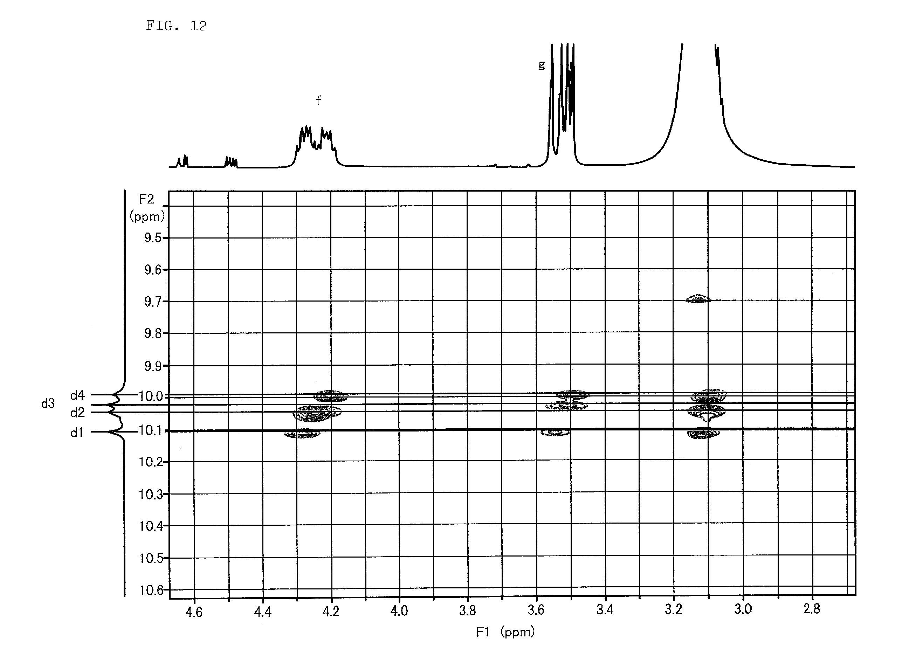

Fig. 12 shows the enlarged NOESY spectrogram observed for the sample 2. There were observed the d proton splitted into four species and therefore, the numbers d1 to d4 were given to these species in the order from the low magnetic field side (in the ascending order). The NOE correlations are summarized as follows:

- There were observed, for the both d1 and d4, the NOE correlations with the both methyl group and -CH₂-CH₂-X;
- There was observed, for d2, the NOE correlation only with -CH₂-CH₂-X;
- There was observed, for d3, the NOE correlation only with methyl group.
The arrangement of side chains as shown in Fig. 1 were elucidated on the basis of the fact that there was not observed any distinct NOE correlation between methyl groups or between -CH₂-CH₂-X groups in addition to the foregoing NOE correlations.

Regarding the numbering of the ¹³C signals and ¹H signals, they were numbered in such a manner that the signals observed in an approximately identical region were numbered in a lump. This is because, the porphyrin skeleton has a repeated structure and therefore, the detailed attribution thereof was quite difficult. As to the number of repetition, there were observed four peaks to be attributable to methyl group (g) and accordingly, the number of repetition would be estimated to be 4.

According to the foregoing procedures, it could be confirmed that the compound had the structure as shown in Fig. 1 in the light of the analytical results of the sample 1 and 2. In this respect, however, it is sufficient that the number of respective side chains and more specifically methyl groups and -CH₂-CH₂-X groups are four in total, the position of each side chain attached may be either one of the 8 sites as shown in Fig. 1 and this is not in contradiction to the foregoing data. Accordingly, the position thereof is not restricted to those specified in Fig. 1.

Then the portion corresponding to X in the group: -CH₂-CH₂-X was investigated.
The product obtained according to the foregoing procedures was subjected to the following analysis:

### (1) Qualitative Analysis According to Pyrolytic GC/MS:

To remove TFA or the like, the sample was heated at 280°C for 10 minutes in a heating furnace, then thermally decomposed at 600 °C and the decomposition products were separated and analyzed using the gas chromatograph/mass spectrograph (hereunder referred to as "GC/MS") as detailed below.

### Name of the Device:

A device available from Agilent Technologies under the trade name of HP5973: Type equipped with a mass-selective detector; and HP6890 Type: Gas Chromatograph;
A device available from FRONTIER LAB under the trade name of PY-2020iD: Heating Furnace type Decomposition Device.
Sample to be Analyzed: A solution (0.5 mL) obtained by adding 1 mL of AcCN to 2 mg of each sample was poured into a sample cup and then AcCN was removed by purging the cup with nitrogen gas.

### (2) Analysis with Electrospray Ionization-Mass Spectrograph:

To examine the molecular weight of the components present in the solution, each sample was analyzed using the Electrospray Ionization-Mass Spectrograph (hereunder referred to as "ESI-MS") as detailed below:
Name of the Device: Qstar available from Applied Biosystems;
Solvent Introduced: AcCN/0.05% aqueous formic acid solution (50/50);
Method of Introduction: The solution to be analyzed was directly
introduced using a 30µL loop.
Mode of Measurement: Positive Mode;
Solution to be Analyzed: A small amount of a solution obtained by adding 1 mL of AcCN to 2 mg of each sample was dispensed in a vial and then diluted to about 100 ppm with the introduction solvent.

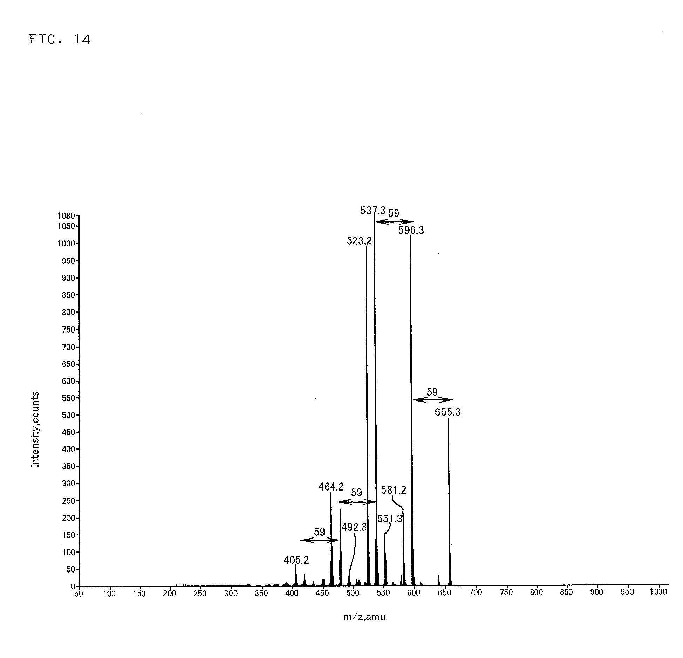

The results obtained in the foregoing GC/MS analysis indicated that carbon dioxide was detected in the sample. In addition, As seen from the results (see Fig. 14) obtained in the foregoing ESI-MS analysis, fragment ions having a molecular weight of 59 (4 peaks) were detected and accordingly, it was confirmed that the fragment comprised an ethyl ester group or acetic acid group (a propionic acid group).

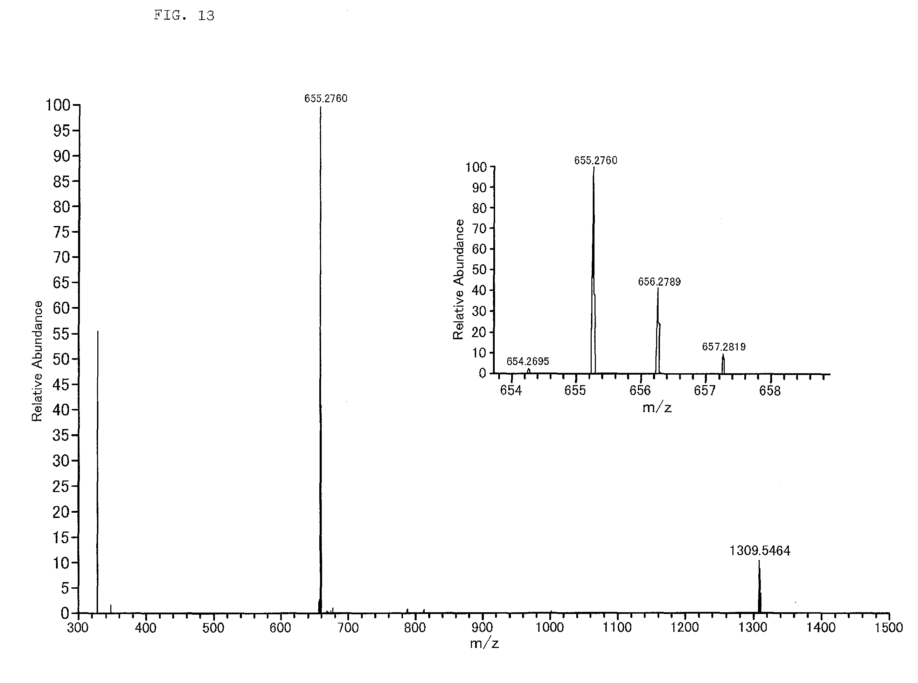

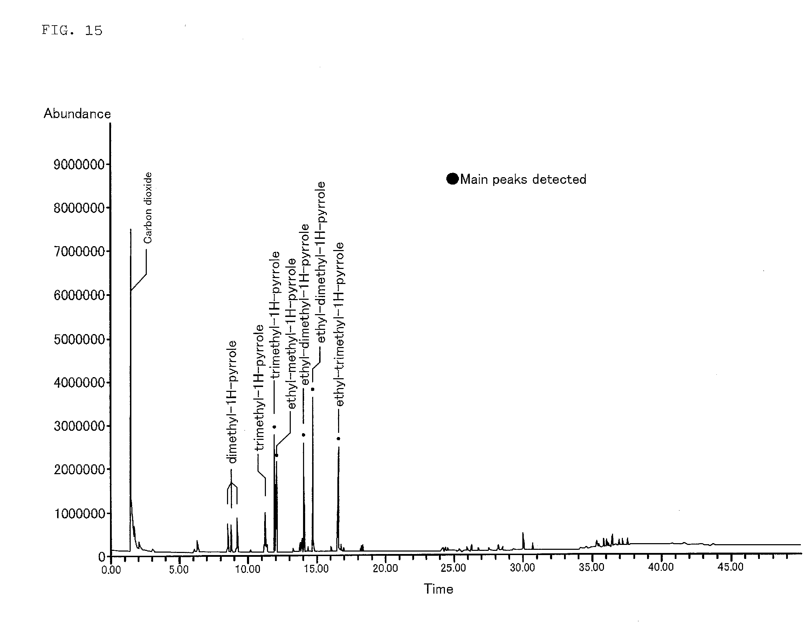

Moreover, the foregoing product was analyzed by the pyrolytic GC-MS technique and as a result, the main component thereof was found to be a pyrrole compound as shown in Fig. 15. This fact could be confirmed by comparing the mass spectral data of each component with the data base and as a result, it could be confirmed that the product had a pyrrole ring structure in the molecule. In addition, in respect of the detailed molecular weight, it was detected as an ion formed through the addition of a hydrogen ion to the product as shown in Fig. 13 and thus the molecular weight thereof was found to be 654.2682 (=655.2760 - 1.0078). From the foregoing, it was confirmed that the product was a porphyrin compound carrying, on the side chains, 4 ethyl ester groups or acetic acid groups (propionic acid groups) and 4 methyl groups, having a molecular weight of 654 and having the following molecular formula: C₃₆H₃₈O₈N₄ and that there was not any transition metal at the center of the porphyrin ring.

### Example 1:

In this Example, lignin used was a high purity product (available from Sigma Company, Catalogue No. 471003 having a molecular weight of 60,000) free of any impurities such as reducing sugar and celluloses. There was introduced 1 mL of a solution containing 2.5 mg/mL of this lignin, 0.05M of KOH and 50µg/mL of the porphyrin carrying 4 carboxyl groups in the molecule and prepared in the foregoing Preparation Example 1 into a cylindrical tube having an optical transmission almost similar to that of an Eppendorf tube of polypropylene and the content of the tube was irradiated, for 12 hours, with UV light rays emitted from an ENF Type UV lamp available from SPECTRONICS Company. Thereafter, the tube was heated to 80°C for 60 minutes, the gas evaporated from the content of the tube was analyzed using GC/MS QP-2010 equipped with a column DB-WAX, available from Shimadzu Corporation and 170µg/mL of methanol was detected. At this stage, there were further detected 140µg/mL of formic acids, 25µg/mL of malic acid, 19µg/mL of acetic acid, 5.4µg/mL of succinic acid, and 6.2µg/mL of pyruvic acid. In this case, it was possible to obtain 6.8% by mass of methanol and 5.6% by mass of formic acid on the basis of the mass of the dry lignin.

Moreover, the same procedures used above were repeated using, as the raw lignin, a high purity product (available from Aldrich Company, Catalogue No. 471046, having a molecular weight of 12,000) free of any impurities such as reducing sugar and celluloses; a product having a slightly low purity (available from Sigma Company, Catalogue No. 471038, molecular weight: 52,000) containing reducing sugar; and a water-insoluble product (available from Aldrich Company, Catalogue No. 370967). As a result, with respect to the amounts of methanol and the foregoing organic acids, almost the same results observed above were obtained. More specifically, the present invention permitted the production and the isolation of alcohols and organic acids starting from lignin, irrespective of the presence of impurities in lignin used as a raw material, the average molecular weight of the lignin used and the solubility thereof in water.

Furthermore, the same procedures used above were repeated except that the solar light rays were substituted for the UV light rays used above and as a result, almost the same results observed above were obtained, with respect to the amounts of methanol and organic acids thus produced.

As has been discussed above, the sample in the form of a reaction liquid prepared by the irradiation with UV light rays was analyzed using a gel filtration column according to the HPLC (high performance liquid chromatography) technique. The solution prior to the irradiation with UV rays as a control was likewise analyzed according to the same method. As a result of the analysis, the lignin present in the reaction solution was detected as an absorbance peak as determined at 310 nm and observed for the fraction eluted at a time ranging from 7 to 9 minutes, for the lignin prior to the UV-irradiation. On the other hand, when a porphyrin was added to lignin and the resulting mixture was irradiated with UV rays to thus convert the same into methanol and organic acids as has been discussed above, the absorbance peak area attributable to the lignin and detected at 310 nm and at an elution time ranging from 7 to 9 minutes was reduced to 20%. In other words, it was found that the addition of a porphyrin and the UV-irradiation could increase the lignin-decomposition rate up to 80%. The resulting decomposition products were eluted as fractions containing components having lower molecular weights according to the gel filtration technique. As a result, about 8.5% by mass of methanol and about 7% by mass of formic acid on the basis of the mass of the decomposed lignin were prepared.

### Example 2:

The lignin used in this Example was a high purity product (available from Sigma Company, Catalogue No. 471003, molecular weight: 60,000) free of any impurities such as reducing sugar and celluloses. There was introduced 1 mL of a solution containing 2.5 mg/mL of this lignin, 0.05M of KOH and, as synthetic porphyrins, 50µg/mL each of Protoporphyrin IX (available from ALDRICH Company) carrying 2 carboxyl groups in the molecule and Uroporphyrin I (available from Sigma Company) carrying 8 carboxyl groups in the molecule or Coproporphyrin I (available from ALDRICH Company) carrying 4 carboxyl groups in the molecule into a cylindrical tube having an optical transmission almost similar to that of an Eppendorf tube of polypropylene and the content of the tube was irradiated, for 12 hours, with UV light rays emitted from an ENF Type UV lamp available from SPECTRONICS Company. Thereafter, the reaction liquid thus obtained was heated to 80°C for 60 minutes, and the gas evaporated from the content of the tube was analyzed using GC/MS QP-2010 equipped with a column DB-WAX, available from Shimadzu Corporation. When repeating the foregoing procedures over several times, there were prepared methanol and organic acids in almost the same amounts as those observed in Example 1 on the basis of the dry mass of the lignin used, in each of the cases which made use of Protoporphyrin IX, Uroporphyrin I and Coproporphyrin I. More specifically, the starting lignin was found to be converted into about 6 to 9% by mass of methanol and about 2 to 4% by mass of formic acid, on the basis of the dry mass of the lignin used.

Each sample collected from each of the foregoing reaction solutions obtained above was analyzed using a gel filtration column according to the HPLC (high performance liquid chromatography) technique. The solution prior to the irradiation with UV rays as a control was likewise analyzed according to the same method. As a result of the analysis, the lignin present in the reaction solution was detected as an absorbance peak as determined at 310 nm and observed for the fraction eluted at a time ranging from 7 to 9 minutes, for the lignin prior to the UV-irradiation. On the other hand, in the case of the reaction solution obtained by adding each of the foregoing porphyrins to lignin and irradiating the lignin with UV rays to thus convert the same into alcohol and organic acids (such as formic acid), the peak area attributable to the lignin was reduced to 60% for the reaction solution obtained using Protoporphyrin IX, to about 15% for the reaction solution obtained using Uroporphyrin I and to about 20% for the reaction solution obtained using Coproporphyrin I. In other words, it was found that the addition of a porphyrin to lignin and the irradiation of the resulting mixture with UV light rays could increase the lignin-decomposition rate up to about 40% in the case of Protoporphyrin IX, about 85% in the case of Uroporphyrin I, and about 80% in the case of Coproporphyrin I. The resulting decomposition products were eluted as fractions containing components having lower molecular weights according to the gel filtration technique.

Moreover, the same procedures used above were repeated using, as the raw lignin, a high purity product (available from Aldrich Company, Catalogue No. 471046, molecular weight: 12,000) free of any impurities such as reducing sugar and celluloses; a product having a slightly low purity (available from Sigma Company, Catalogue No. 471038, molecular weight: 52,000) containing reducing sugar; and a water-insoluble product (available from Aldrich Company, Catalogue No. 370967). As a result, with respect to the decomposition of lignin, it was found that almost the same results were obtained even when using the synthetic porphyrins. More specifically, the present invention permitted the decomposition of lignin to almost the same extent, irrespective of the presence of impurities in lignin used as a raw material, the average molecular weight of the lignin used and the solubility thereof in water.

Furthermore, the same procedures used above were repeated except that the solar light rays were substituted for the UV light rays used above and as a result, it was found that almost the same results were obtained.

### Example 3:

In this Example, lignin used was a high purity product (available from Sigma Company, Catalogue No. 471003, molecular weight: 60,000) free of any impurities such as reducing sugar and celluloses. There was introduced 1 mL of a solution containing 1. 8mg/mL of this lignin, 0.05M of KOH and 50µg/mL of Protoporphyrin IV available from Sigma Company (Catalogue No. 258385-1G) into a cylindrical tube having an optical transmission almost similar to that of an Eppendorf tube of polypropylene and the content of the tube was irradiated, for 12 hours, with UV light rays emitted from an ENF Type UV lamp available from SPECTRONICS Company. Thereafter, the tube was heated to 80°C for 60 minutes, the gas evaporated from the content of the tube was analyzed using GC/MS QP-2010 equipped with a column DB-WAX, available from Shimadzu Corporation and 54µg/mL of methanol was detected. In other words, it was possible to obtain 3.0% by mass of methanol on the basis of the mass of the dry lignin.

Moreover, the same procedures used above were repeated using, as the raw lignin, a high purity product (available from Aldrich Company, Catalogue No. 471046, molecular weight: 12,000) free of any impurities such as reducing sugar and celluloses; a product having a slightly low purity (available from Sigma Company, Catalogue No. 471038, molecular weight: 52,000) containing reducing sugar; and a water-insoluble product (available from Aldrich Company, Catalogue No. 370967). As a result, with respect to the amount of methanol, almost the same results as those observed above were obtained. More specifically, the present invention permitted the production and the isolation of alcohols and organic acids starting from lignin, irrespective of the presence of impurities in lignin used as a raw material, the average molecular weight of the lignin used and the solubility thereof in water.

Furthermore, the same procedures used above were repeated except that the solar light rays were substituted for the UV light rays used above and as a result, it was found that almost the same results were obtained, with respect to the amount of methanol formed.

### Example 4:

In this Example, lignin used was a high purity product (available from Sigma Company, Catalogue No. 471003, molecular weight: 60,000) free of any impurities such as reducing sugar and celluloses. There was introduced 1 mL of a solution containing 7.5 mg/mL of this lignin and 0.05M of KOH into a cylindrical tube having an optical transmission almost similar to that of an Eppendorf tube of polypropylene and the content of the tube was heated to 80°C for 60 minutes, the gas evaporated from the tube was analyzed using GC/MS QP-2010 equipped with a column DB-WAX, available from Shimadzu Corporation and as a result, 93µg/mL of methanol was detected. In other words, it was possible to obtain 1.24% by mass of methanol on the basis of the mass of the dry lignin.

Moreover, the same procedures used above were repeated using, as the raw lignin, a high purity product (available from Aldrich Company, Catalogue No. 471046, molecular weight: 12, 000) free of any impurities such as reducing sugar and celluloses and a water-insoluble product (available from Aldrich Company, Catalogue No. 370967). As a result, with respect to the amount of methanol, almost the same results observed above were obtained. More specifically, the present invention permitted the production of almost the same amount of methanol starting from lignin, irrespective of the presence of impurities in lignin used as a raw material, the average molecular weight of the lignin used and the solubility thereof in water.

### Example 5:

In this Example, lignin used was a high purity product (available from Sigma Company, Catalogue No. 471003, molecular weight: 60,000) free of any impurities such as reducing sugar and celluloses. There was introduced 1 mL of a solution containing 7.5 mg/mL of this lignin and 0.05M of KOH into a cylindrical tube having an optical transmission almost similar to that of an Eppendorf tube of polypropylene and the content of the tube was irradiated, for 24 hours, with UV light rays emitted from an ENF Type UV lamp available from SPECTRONICS Company. Thereafter, the content of the tube was heated to 80°C for 60 minutes, the gas evaporated from the tube was analyzed using GC/MS QP-2010 equipped with a column DB-WAX, available from Shimadzu Corporation and as a result, 150µg/mL of methanol was detected. In other words, it was possible to obtain 2% by mass of methanol on the basis of the mass of the dry lignin.

Moreover, the same procedures used above were repeated using, as the raw lignin, a high purity product (available from Aldrich Company, Catalogue No. 471046, molecular weight: 12,000) free of any impurities such as reducing sugar and celluloses and a water-insoluble product (available from Aldrich Company, Catalogue No. 370967). As a result, with respect to the amount of methanol, almost the same results were obtained. More specifically, the present invention permitted the production of almost the same amount of methanol starting from lignin, irrespective of the presence of impurities in lignin used as a raw material, the average molecular weight of the lignin used and the solubility thereof in water.

Furthermore, the same procedures used above were repeated except that the solar light rays were substituted for the UV light rays used above and as a result, it was found that almost the same results observed above were obtained, with respect to the amount of methanol prepared.

### Example 6:

In this Example, lignin used was a high purity product (available from Sigma Company, Catalogue No. 471003, molecular weight: 60,000) free of any impurities such as reducing sugar and celluloses. There was introduced 1 mL of a solution containing 2.5 mg/mL of this lignin, 0.05M of KOH and 25µg/mL of the porphyrin prepared in the foregoing Preparation Example 1 into a cylindrical tube having an optical transmission almost similar to that of an Eppendorf tube of polypropylene and the content of the tube was irradiated, for 12 hours, with UV light rays emitted from an ENF Type UV lamp available from SPECTRONICS Company. Thereafter, the content of the tube was heated to 80°C for 60 minutes, the gas evaporated from the tube was analyzed using GC/MS QP-2010 equipped with a column DB-WAX, available from Shimadzu Corporation and as a result, 160µg/mL of methanol was detected. In other words, it was possible to obtain 6.4% by mass of methanol on the basis of the mass of the dry lignin. These results clearly indicate that the addition of a porphyrin as a photocatalyst to the lignin-alkaline compound-containing solution would permit the significant increase of methanol produced from lignin.

Moreover, the same procedures used above were repeated using, as the raw lignin, a high purity product (available from Aldrich Company, Catalogue No. 471046, molecular weight: 12,000) free of any impurities such as reducing sugar and celluloses and a water-insoluble product (available from Aldrich Company, Catalogue No. 370967). As a result, with respect to the amount of methanol, almost the same results were obtained. More specifically, the present invention permitted the production of almost the same amount of methanol starting from lignin, irrespective of the presence of impurities in lignin used as a raw material, the average molecular weight of the lignin used and the solubility thereof in water.

Furthermore, the same procedures used above were repeated except that the solar light rays were substituted for the UV light rays used above and as a result, it was found that almost the same results were obtained, with respect to the amount of methanol formed.

### Example 7:

In this Example, lignin used was a high purity product (available from Sigma Company, Catalogue No. 471003, molecular weight: 60,000) free of any impurities such as reducing sugar and celluloses. There was introduced 1 mL of a solution containing 2.5 mg/mL of this lignin into a cylindrical tube having an optical transmission approximately equal to that of an Eppendorf tube of polypropylene and the content of the tube was irradiated, for 24 hours, with UV light rays emitted from an ENF Type UV lamp available from SPECTRONICS Company. Thereafter, the content of the tube was heated to 80°C for 60 minutes, the gas evaporated from the tube was analyzed using GC/MS QP-2010 equipped with a column DB-WAX, available from Shimadzu Corporation and as a result, 21µg/mL of methanol was detected. In other words, it was possible to obtain 0.84% by mass of methanol on the basis of the mass of the dry lignin.

Moreover, the same procedures used above were repeated using, as the raw lignin, a high purity product (available from Aldrich Company, Catalogue No. 471046, molecular weight: 12, 000) free of any impurities such as reducing sugar and celluloses and a water-insoluble product (available from Aldrich Company, Catalogue No. 370967). As a result, with respect to the amount of methanol, almost the same results observed above were obtained. More specifically, the present invention permitted the production of almost the same amount of methanol starting from lignin, irrespective of the presence of impurities in lignin used as a raw material, the average molecular weight of the lignin used and the solubility thereof in water.

Furthermore, the same procedures used above were repeated except that the solar light rays were substituted for the UV light rays used above and as a result, it was found that almost the same results were obtained, with respect to the amount of methanol.

### Example 8:

In this Example, the aromatic hydrocarbon-decomposition method according to the present invention will be described below in detail. In this respect, the aromatic hydrocarbon is one in which an oxygen atom is bonded to the benzene ring thereof.

The aromatic hydrocarbon, in which an oxygen atom is bonded to the benzene ring thereof, herein used was Remasol Brilliant Blue (RBBR available from Sigma Company) and the aromatic hydrocarbon was subjected to the photolytic decomposition. This RBBR is a compound similar to dioxins and has been used as an indicator reagent with respect to the decomposition of dioxins.

There were introduced 1mL of a solution containing 250µg/mL of RBBR, 0.05M of KOH, and 25µg/mL each of a porphyrin prepared in the foregoing Preparation Example 1; Protoporphyrin IX (available from Aldrich Company); Uroporphyrin I (available from Sigma Company) ; and Coproporphyrin I (available from Aldrich Company) (the solvent used : 30% acetonitrile, 0.1% trifluoroacetic acid, 30% methanol) into a cylindrical tube having an optical transmission approximately equal to that of an Eppendorf tube of polypropylene and the content of the tube was irradiated, for 2 hours, with UV light rays emitted from an ENF Type UV lamp available from SPECTRONICS Company. As a result, it was found that the bluish color tone of RBBR disappeared irrespective of the kinds of porphyrins used and the solvents used. This clearly indicates that RBBR is decomposed.

Furthermore, the same procedures used above were repeated except that the solar light rays were substituted for the UV light rays used above and as a result, it could be confirmed that RBBR was likewise decomposed.

### Example 9:

In this Example, there were used xylene cyanol (available from Takara Co., Ltd.) and Bromophenol Blue (available from Takara Co., Ltd.) as examples of the aromatic hydrocarbon compounds in which an oxygen atom was linked to the benzene ring of the compound and these compounds were subjected to photolytic decomposition.

There was introduced 1 mL of a solution containing 5 mg/mL of xylene cyanol and 5 mg/mL of Bromophenol Blue, 0.05M of KOH, and 4 0µg/mL each of a porphyrin prepared according to the method disclosed in Preparation Example 1, Protoporphyrin IX (available from Aldrich Company); Uroporphyrin I (available from Sigma Company); Coproporphyrin I (available from Aldrich Company); and Ethioporphyrin (available from Aldrich Company) (as the solvent used, 30%acetonitrile, 0.1% trifluoroacetic acid), into a cylindrical tube having an optical transmission approximately equal to that of an Eppendorf tube of polypropylene and the content of the tube was irradiated, for 2 hours, with UV light rays emitted from an ENF Type UV lamp available from SPECTRONICS Company. As a result, it was found that the bluish and yellowish color tones of xylene cyanol and Bromophenol Blue disappeared irrespective of the kinds of porphyrins used and the kinds of solvents used. This clearly indicates that xylene cyanol and Bromophenol Blue can be decomposed.

Furthermore, the same procedures used above were repeated except that the solar light rays were substituted for the UV light rays used above and as a result, it could be confirmed that xylene cyanol and Bromophenol Blue was likewise decomposed.

### Example 10:

In this Example, there was described the method, according to the present invention, for making hydrogen ions release from lignin which making use of the lignin decomposition catalyst of the present invention. Used in this Example as such lignin was a high purity product (available from Sigma Company, Catalogue No. 471003, molecular weight: 60,000) free of any impurities such as reducing sugar and celluloses.

There was introduced 1 mL of a solution containing 2.5 mg/mL of this lignin and 50µg/mL of a porphyrin prepared according to the method disclosed in Preparation Example 1, into a cylindrical tube having an optical transmission approximately equal to that of an Eppendorf tube of polypropylene and the content of the tube was irradiated, for 12 hours, with UV light rays emitted from an ENF Type UV lamp available from SPECTRONICS Company. As a result, it was found that the pH value of the lignin-containing reaction solution was reduced from 9.2 to 6.4. This clearly indicates that hydrogen ions are released from lignin.

Moreover, the same procedures used above were repeated using, as the raw lignin, a high purity product (available from Aldrich Company, Catalogue No. 471046, molecular weight: 12,000) free of any impurities such as reducing sugar and celluloses; a product having a slightly low purity (available from Sigma Company, Catalogue No. 471038, molecular weight: 52,000) containing reducing sugar; and a water-insoluble product (available from Aldrich Company, Catalogue No. 370967). As a result, it was found that hydrogen ions are likewise released from lignin. More specifically, the present invention permitted the release of hydrogen ions in the same extent starting from lignin, irrespective of the presence of impurities in lignin used as a raw material, the average molecular weight of the lignin used and the solubility thereof in water.

Furthermore, the same procedures used above were likewise repeated except that the solar light rays were substituted for the UV light rays used above and as a result, it was found that almost the same results were obtained.

### Example 11:

Used in this Example as lignin was a high purity product (available from Aldrich Company, Catalogue No. 471003, molecular weight: 60,000) free of any impurities such as reducing sugar and celluloses. There was introduced 1 mL of a solution containing 2.5 mg/mL of this lignin, 2.5 mM of KOH and 50µg/mL of a porphyrin prepared according to the method disclosed in Preparation Example 1, into a cylindrical tube having an optical transmission approximately equal to that of an Eppendorf tube of polypropylene and the content of the tube was irradiated, for 12 hours, with UV light rays emitted from an ENF Type UV lamp available from SPECTRONICS Company. The sample was analyzed using a gel-filtration column according to HPLC (High Performance Liquid Chromatography). The solution prior to the UV-irradiation as a control was likewise analyzed by the same method. As a result of the analysis, the lignin present in the reaction solution was detected as an absorbance peak as determined at a detection wavelength of 310 nm and observed for the fraction eluted at a time ranging from 7 to 9 minutes, for the lignin prior to the UV-irradiation. On the other hand, when a porphyrin was added to lignin and the latter was irradiated with UV rays, the absorption peak area attributable to the lignin and detected at 310 nm and at an elution time ranging from 7 to 9 minutes was reduced to 78%. In other words, it was found that 22% of the lignin was decomposed by the addition of a porphyrin to lignin and the UV-irradiation. Moreover, it was found that the pH value of the lignin-containing reaction solution was reduced from 10.7 to 6.2. This clearly indicates that hydrogen ions are released from lignin.

Moreover, the same procedures used above were repeated using, as the raw lignin, a high purity product (available from Aldrich Company, Catalogue No. 471046, molecular weight: 12,000) free of any impurities such as reducing sugar and celluloses; a product having a slightly low purity (available from Sigma Company, Catalogue No. 471038, molecular weight: 52,000) containing reducing sugar; and a water-insoluble product (available from Aldrich Company, Catalogue No. 370967). As a result, it was found that the same results observed above could be obtained. More specifically, the present invention permitted the release of hydrogen ions in the same extent observed above starting from lignin, irrespective of the presence of impurities in lignin used as a raw material, the average molecular weight of the lignin and the solubility thereof in water.

Furthermore, the same procedures used above were likewise repeated except that the solar light rays were substituted for the UV light rays used above and as a result, it was found that almost the same results were obtained.

### Example 12:

In this Example, there was described the method, according to the present invention, for making hydrogen ions release from lignin which making use of the lignin decomposition catalyst of the present invention. Used in this Example as such lignin was a product having a slightly low purity (available from Sigma Company, Catalogue No. 471038, molecular weight: 52,000) containing reducing sugar.

There was introduced 1 mL of a solution containing 5 mg/mL of this lignin and 50µg/mL of a porphyrin prepared according to the method disclosed in Preparation Example 1, into a cylindrical tube having an optical transmission approximately equal to that of an Eppendorf tube of polypropylene and the content of the tube was irradiated, for 12 hours, with UV light rays emitted from an ENF Type UV lamp available from SPECTRONICS Company. As a result, it was found that the pH value of the lignin-containing reaction solution was reduced from 6.2 to 4.8. This clearly indicates that hydrogen ions are released from lignin.

Moreover, the same procedures used above were repeated using, as the raw lignin, a high purity product (a product available from Sigma Company, Catalogue No. 471003, molecular weight: 60,000 and a product available from Aldrich Company, Catalogue No. 471046, molecular weight: 12,000) free of any impurities such as reducing sugar and celluloses; and a water-insoluble product (available from Aldrich Company, Catalogue No. 370967). As a result, it was found that the same results observed above could be obtained. More specifically, the present invention permitted the release of hydrogen ions in the same extent observed above starting from lignin, irrespective of the presence of impurities in lignin used as a raw material, the average molecular weight of the lignin and the solubility thereof in water.

Furthermore, the same procedures used above were likewise repeated except that the solar light rays were substituted for the UV light rays used above and as a result, it was found that almost the same results were obtained.

### Example 13:

In this Example, there was described the method, according to the present invention, for making hydrogen ions release from lignin which making use of the lignin decomposition catalyst of the present invention. Used in this Example as such lignin was a high purity product (available from Sigma Company, Catalogue No. 471003, molecular weight: 60,000) free of any impurities such as reducing sugar and celluloses.

There was introduced 1 mL of a solution containing 2.5 mg/mL of this lignin, 2.5 mM of KOH and 50µg/mL of a porphyrin (Protoporphyrin IV available from Sigma Company, Catalogue No. 258385-1G), into a cylindrical tube having an optical transmission approximately equal to that of an Eppendorf tube of polypropylene and the content of the tube was irradiated, for 12 hours, with UV light rays emitted from an ENF Type UV lamp available from SPECTRONICS Company. As a result, it was found that the pH value of the lignin-containing reaction solution was reduced from 10.7 to 7.4. This clearly indicates that hydrogen ions are released from lignin.

Furthermore, the same procedures used above were likewise repeated except that the solar light rays were substituted for the UV light rays used above and as a result, it was found that almost the same results were obtained.

### Example 14:

Used in this Example, as lignin, was a high purity product (available from Sigma Company, Catalogue No. 471003, molecular weight: 60,000) free of any impurities such as reducing sugar and celluloses. There was introduced 1 mL of a solution containing 2.5 mg/mL of this lignin, 0.05 mM of KOH and 50µg/mL of a porphyrin prepared according to the method disclosed in Preparation Example 1, into a cylindrical tube having an optical transmission approximately equal to that of an Eppendorf tube of polypropylene and the content of the tube was irradiated, for 12 hours, with UV light rays emitted from an ENF Type UV lamp available from SPECTRONICS Company. The sample thus obtained was analyzed using a gel-filtration column according to the HPLC technique. The solution prior to the UV-irradiation as a control was likewise analyzed by the same method. As a result of the comparison with the results observed for the sample prior to the UV-irradiation, 72% of the lignin was decomposed in the sample after the UV-irradiation, in the light of the absorption peak area attributable to the lignin and detected at the detection wavelength of 310 nm and at an elution time of 7 minutes. The resulting decomposition products were eluted as fractions containing components having lower molecular weights according to the gel filtration technique. The decomposition products derived from lignin in the method of the present invention show low absorbances and therefore, they could not detected by the use of a detection wavelength of 310 nm. However, the non-volatile decomposition products present in the eluted samples or fractions were lyophilized and it was found that the decomposition products were recovered as precipitates.

The same procedures used above were repeated using, as lignin, a high purity product (available from Aldrich Company, Catalogue No. 471046, molecular weight: 12,000) free of any impurities such as reducing sugar and celluloses and a water-insoluble product (available from Aldrich Company, Catalogue No. 370967) and as a result, almost the same results observed above were obtained. More specifically, the addition of a porphyrin as a photocatalyst to the lignin-alkali compound-containing solution provided almost the same result observed above, with respect to the photolytic decomposition of lignin, irrespective of the presence of impurities in lignin used as a raw material, the average molecular weight of the lignin and the solubility thereof in water.

Furthermore, the same procedures used above were likewise repeated except that the solar light rays were substituted for the UV light rays used above and as a result, it was found that almost the same results were obtained.

### Example 15:

Used in this Example as lignin was a high purity product (available from Sigma Company, Catalogue No. 471003, molecular weight: 60,000) free of any impurities such as reducing sugar and celluloses. There was introduced 0.5 mL of a solution containing 205mg/mL of this lignin, 0.05M of KOH and 50µg/mL of coproporphyrin I (available from ALDRICH Company) carrying 4 carboxyl groups in the molecule as a synthetic porphyrin, into a 2 mL volume cylindrical tube having an optical transmission approximately equal to that of an Eppendorf tube of polypropylene, while remaining a space for the air and the content of the tube was irradiated, for 48 hours, with UV light rays emitted from an ENF Type UV lamp available from SPECTRONICS Company. The reaction solution thus obtained was treated by the same procedures used in Example 2. As a result, it was confirmed that not less than 80% of the lignin was decomposed.

In addition, oxygen gas was introduced into the space for the air and then the content of the tube was irradiated with UV light rays in the same manner used above. As a result, it was likewise confirmed that not less than 80% of the lignin was decomposed.

In addition, nitrogen gas was introduced into the space for the air and then the content of the tube was irradiated with UV light rays in the same manner used above. As a result, it was confirmed that about 10% of the lignin was decomposed.

Moreover, the foregoing porphyrin-containing lignin solution was introduced into an Eppendorf tube in such an amount that any space for the air did not remain in the tube, the lignin solution was then irradiated with UV light rays in the same manner used above and as a result, it was recognized that any decomposition of lignin was not detected.

In addition, the same procedures used above were repeated using, as lignin, a high purity product (available from Aldrich Company, Catalogue No. 471046, molecular weight: 12,000) free of any impurities such as reducing sugar and celluloses; a product having a slightly low purity (available from Sigma Company, Catalogue No. 471038, molecular weight: 52,000) containing reducing sugar; and a water-insoluble product (available from Aldrich Company, Catalogue No. 370967) and as a result, almost the same results observed above were obtained with respect to the decomposition of lignin. More specifically, the method of present invention permitted the decomposition of lignin to almost the same extent observed above, irrespective of the presence of impurities in lignin used as a raw material, the average molecular weight of the lignin and the solubility thereof in water.

Furthermore, the same procedures used above were likewise repeated except that the solar light rays were substituted for the UV light rays used above and as a result, it was found that almost the same results observed above were obtained with respect to the decomposition of lignin.

### [Industrial Applicability]

The lignin decomposition catalyst according to the present invention is not an enzyme derived from, for instance, white rot-causal bacteria and it is a non-proteinous catalyst. The use of this catalyst would permit the decomposition of lignin mainly by the aid of optical energy to thus isolate and separate alcohols such as methanol and organic acids such as formic acid and to release hydrogen ions from the lignin as a non-effective natural resource which constitutes 20 to 30% of the wood, according to a simple method. Accordingly, the lignin decomposition catalyst of the present invention would permit the expansion of the fields of applications of lignin whose decomposition within the natural world is quite difficult. More specifically, the alcohols derived from lignin can be used in the field of the fuel industry as a substitute fuel for fossil fuels such as gasoline, the organic acids likewise derived from lignin can be used in a variety of industrial fields and the hydrogen ions derived from lignin can be used in, for instance, fuel batteries or can be used in the fields of the electric power industries.

In addition, the aromatic hydrocarbon-decomposition catalyst according to the present invention can decompose aromatic hydrocarbons each carrying an oxygen atom linked to the benzene ring thereof and accordingly, the use thereof would permit the treatment of harmful industrial waste and the purification of contaminated soil and the ground surface. Thus, the catalyst according to the present invention can be used in the fields of the treatment of industrial waste and the field of the purification of soil or the like.

## Claims

1. A catalyst for the decomposition of lignin comprising a porphyrin which can show its catalytic function through the irradiation thereof with light rays.

2. A catalyst for the decomposition of lignin comprising a porphyrin which can show its catalytic function in an alkaline solution.

3. A catalyst for the decomposition of lignin comprising a porphyrin which can show its catalytic function in an alkaline solution, while irradiating the same with light rays.

4. The catalyst for the decomposition of lignin as set forth in any one of claims 1 to 3, wherein the porphyrin is a tetrapyrrole compound carrying a methyl group and an ethyl ester group or an acetate group (propionic acid group) on the porphyrin ring.

5. The catalyst for the decomposition of lignin as set forth in any one of claims 1 to 3, wherein the porphyrin is a tetrapyrrole compound carrying four methyl groups and four ethyl ester groups or acetate groups (propionic acid groups) on the porphyrin ring.

6. The catalyst for the decomposition of lignin as set forth in any one of claims 1 to 3, wherein the porphyrin is one having, in the molecule, a carboxyl group.

7. The catalyst for the decomposition of lignin as set forth in claim 6, wherein the porphyrin is one having, in the molecule, two, four or eight carboxyl groups, in total.

8. The catalyst for the decomposition of lignin as set forth in claim 6, wherein the porphyrin is at least one member selected from the group consisting of uroporphyrin, protoporphyrin and coproporphyrin.

9. The catalyst for the decomposition of lignin as set forth in any one of claims 1 to 3, wherein the porphyrin is a tetrapyrrole compound having a porphyrin ring structure, which is obtained by cultivating *Escherichia coli* in a culture medium and then isolating the same from the medium.

10. The catalyst for the decomposition of lignin as set forth in any one of claims 1 to 3, wherein the catalyst comprises a culture medium containing a tetrapyrrole compound having a porphyrin ring structure, which is obtained by cultivating *Escherichia coli* in a culture medium.

11. The catalyst for the decomposition of lignin as set forth in claim 9 or 10, wherein the *Escherichia coli* is one which cannot express the gene ypjD (b2611) due to the variation thereof.

12. The catalyst for the decomposition of lignin as set forth in any one of claims 9 to 11, wherein the *Escherichia coli* is an insertion variant in which the transposon for the gene ypj D (b2611) is inserted.

13. A method for the preparation of alcohols and organic acids comprising the steps of adding an alkaline compound-containing solution to lignin and then separating and isolating alcohols and organic acids released from the resulting solution containing the lignin and the alkaline compound.

14. The method for the preparation of alcohols and organic acids as set forth in claim 13, wherein after the irradiation of the solution containing lignin and the alkaline compound with light rays, the alcohols and organic acids released from the solution containing the lignin and the alkaline compound are separated and isolated.

15. The method for the preparation of alcohols and organic acids as set forth in claim 14, wherein the step for the irradiation with light rays is carried out by the irradiation with ultraviolet rays or solar rays.

16. The method for the preparation of alcohols and organic acids as set forth in claim 13, wherein the alcohols and organic acids released from the solution containing lignin and the alkaline compound are separated and isolated, after further acting a catalyst for the decomposition of lignin as set forth in any one of claims 1 to 12 on the solution containing lignin and the alkaline compound.

17. The method for the preparation of alcohols and organic acids as set forth in claim 16, wherein the alcohols and organic acids released from the solution containing lignin and the alkaline compound are separated and isolated, after acting a catalyst for the decomposition of lignin as set forth in any one of claims 1 to 12 on the solution containing lignin and the alkaline compound and further irradiating the resulting mixture with light rays.

18. The method for the preparation of alcohols and organic acids as set forth in claim 17, wherein the step for the irradiation with light rays is carried out by the irradiation with ultraviolet rays or solar rays.

19. The method for the preparation of alcohols and organic acids as set forth in any one of claims 13 to 18, wherein the alkaline compound is at least one member selected from the group consisting of KOH and NaOH.

20. A method for the preparation of alcohols and organic acids comprising the steps of acting a catalyst for the decomposition of lignin as set forth in any one of claims 1 to 12 on lignin and then separating and isolating alcohols and organic acids released from the lignin treated with the catalyst.

21. A method for the preparation of alcohols and organic acids comprising the steps of irradiating lignin with light rays and then separating and isolating alcohols and organic acids released from the lignin irradiated with the light rays.

22. A method for the preparation of alcohols and organic acids, which comprises the steps of irradiating lignin with light rays and then making alcohols and organic acids release from the light-irradiated lignin, wherein a catalyst for the decomposition of lignin as set forth in any one of claims 1 to 10 is acted on lignin, then the lignin is irradiated with light rays and separating and isolating the alcohols and organic acids released from the lignin.

23. The method for the preparation of alcohols and organic acids as set forth in claim 21 or 22, wherein the step for the irradiation with light rays is carried out by the irradiation with ultraviolet rays or solar rays.

24. The method for the preparation of alcohols and organic acids as set forth in any one of claims 13 to 23, wherein the alcohol is methanol; the organic acids are formic acid, acetic acid, malic acid, succinic acid and pyruvic acid.

25. The method for the preparation of alcohols and organic acids as set forth in any one of claims 13 to 24, wherein the separation and isolation of the alcohols is carried out by the distillation.

26. A method for the preparation of lignin decomposition products comprising the step of recovering the lignin decomposition products generated during the separation of the alcohols and organic acids according to the method for the preparation of alcohols and organic acids as set forth in any one of claims 13 to 25.

27. A catalyst for the decomposition of an aromatic hydrocarbon in which an oxygen atom is bonded to a carbon atom constituting the benzene ring thereof, **characterized in that** it comprises a porphyrin.

28. The catalyst for the decomposition of an aromatic hydrocarbon as set forth in claim 27, wherein the porphyrin is a tetrapyrrole compound having a porphyrin ring structure, which is obtained by cultivating *Escherichia coli* in a culture medium and then isolating the compound from the medium.

29. The catalyst for the decomposition of an aromatic hydrocarbon as set forth in claim 27, wherein the catalyst comprises a culture medium containing a tetrapyrrole compound having a porphyrin ring structure, which is obtained by cultivating *Escherichia coli* in a culture medium.

30. The catalyst for the decomposition of an aromatic hydrocarbon as set forth in claim 28 or 29, wherein the *Escherichia coli* is one which cannot express the gene ypjD (b2611) due to the variation thereof.

31. The catalyst for the decomposition of an aromatic hydrocarbon as set forth in any one of claims 28 to 30, wherein the *Escherichia coli* is an insertion variant in which the transposon for the gene ypjD (b2611) is inserted.

32. The catalyst for the decomposition of an aromatic hydrocarbon as set forth in any one of claims 27 to 31, wherein the porphyrin is a tetrapyrrole compound carrying a methyl group and an ethyl ester group or an acetate group (propionic acid group) on the porphyrin ring.

33. The catalyst for the decomposition of an aromatic hydrocarbon as set forth in any one of claims 27 to 32, wherein the porphyrin is a tetrapyrrole compound carrying four methyl groups and four ethyl ester groups or acetate groups (propionic acid groups) on the porphyrin ring.

34. The catalyst for the decomposition of an aromatic hydrocarbon as set forth in claim 27, wherein the porphyrin is at least one member selected from the group consisting of uroporphyrin, protoporphyrin, coproporphyrin and ethioporphyrin.

35. The catalyst for the decomposition of an aromatic hydrocarbon as set forth in claim 27, wherein the porphyrin is one having, in the molecule, a carboxyl group.

36. The catalyst for the decomposition of an aromatic hydrocarbon as set forth in claim 35, wherein the porphyrin is one carrying, in the molecule, two, four or eight carboxyl group in total.

37. The catalyst for the decomposition of an aromatic hydrocarbon as set forth in any one of claims 27 to 36, wherein the aromatic hydrocarbons are dioxins.

38. A method for making hydrogen ions release comprising the steps of acting a lignin decomposition catalyst as set forth in any one of claims 1 to 11 on lignin or a solution containing lignin and an alkaline compound, irradiating the solution with light rays to thus make hydrogen ions release therefrom.

39. Porphyrin having a catalytic function of converting lignin into alcohols and organic acids.

40. Porphyrin **characterized in that** it has a catalytic function of decomposing a compound containing an aromatic hydrocarbon in which an oxygen atom is bonded to a carbon atom constituting the benzene ring thereof.

41. Porphyrin obtained by cultivating the *Escherichia coli* which cannot express the gene ypjD (b2611) due to the variation thereof.

42. A catalyst for the decomposition of lignin comprising the porphyrin obtained by cultivating the *Escherichia coli* which cannot express the gene ypjD (b2611) due to the mutation variation thereof.
